# EUROPÄISCHE PATENTSCHRIFT

(11) **EP 2 892 451 B1**
(45) Veröffentlichungstag und Bekanntmachung des Hinweises auf die Patenterteilung: **29.05.2019**
(21) Anmeldenummer: 13750588.9
(22) Anmeldetag: 21.08.2013
(51) Int. Cl.: A61B 17/70, A61B 17/80, A61B 17/84, A61B 17/86

(54) **BECKENRINGIMPLANTAT**
PELVIC RING IMPLANT
IMPLANT DE LA CEINTURE PELVIENNE

(30) Priorität: 05.09.2012 DE 202012103384 U
(43) Veröffentlichungstag der Anmeldung: 15.07.2015
(73) Patentinhaber: Signus Medizintechnik GmbH, 63755 Alzenau (DE)
(72) Erfinder: EHLER, Sabrina, 63840 Hausen (DE); HARWARTH, Alexander, 63755 Alzenau (DE); OBERMEIER, Frank, 64646 Heppenheim (DE); SIEDLER, Uwe, 63755 Alzenau (DE)
(74) Vertreter: Lippert Stachow Patentanwälte Rechtsanwälte
(86) Internationale Anmeldenummer: PCT/EP2013/067379
(87) Internationale Veröffentlichungsnummer: WO 2014/037220

(56) Entgegenhaltungen:
- US-A- 5 108 397
- US-A1- 2012 095 560
- US-B1- 6 517 541

## Beschreibung

Die Erfindung betrifft ein Implantat zur Stabilisierung von Beckenringfrakturen sowie degenerativen Veränderungen und Instabilitäten und Arthrosen des Iliosakralgelenks.

Der Beckenring umfasst das Sacrum (os sacrum bzw. Kreuzbein) und zwei Hüftbeine (ossa coxae), wobei die beiden Hüftbeine das Sacrum seitlich umfassen. Ein Hüftbein umfasst als größten Knochen das Ilium (os ilium bzw. Darmbein) und daneben kleinere Knochen wie etwa das Sitzbein (os ischii) und das Schambein (os pubis). Das Ilium ist über das Iliosakralgelenk mit dem Sacrum verbunden, wobei das Iliosakralgelenk nur wenig beweglich ist.

Bei einem Beckenringbruch, der beispielsweise bei schweren Stürzen oder Verkehrsunfällen häufig vorkommt, können Frakturen in den Knochen des Beckenrings, wie etwa dem Ilium oder dem Sacrum, sowie auch in dem Iliosakralgelenk auftreten. Früher wurden solche Beckenringfrakturen nicht operativ behandelt, sondern das Zusammenwachsen der Bruchstellen im Beckenring wurde lediglich durch Ruhigstellung gefördert. Da dadurch jedoch häufig keine zufriedenstellende Heilung erreicht werden konnte, haben sich in neuerer Zeit verschiedene Operationstechniken zur Stabilisierung von Beckenringfrakturen etabliert. Bei solchen Stabilisierungen geht es vornehmlich darum, Knochen an beiden Seiten der Bruchstelle zueinander zu fixieren, um ein besseres Zusammenwachsen der Knochen zu ermöglichen. Dabei wird auch angestrebt, die Knochen in ihrer ursprünglichen Stellung zu fixieren, damit nach der Heilung eines Bruchs keine bleibenden Schäden zurückbleiben. Bei den Operationstechniken zur Stabilisierung von Beckenringfrakturen kommen herkömmlicherweise Implantate zum Einsatz. Beispielsweise sind als Implantate Knochenschrauben bekannt, mit denen Knochen gegeneinander verschraubt werden. Die Schrauben können beispielsweise einen Schraubenkopf und ein Knochengewinde aufweisen, sodass das Knochengewinde durch die Knochen geschraubt werden kann und der Schraubenkopf einen Anpressdruck auf zwei Knochenfragmente entlang des Bruchs erzeugt. Ebenso sind als Implantate Schrauben bekannt, die an einem Ende ein Knochengewinde aufweisen und an dem anderen Ende ein metrisches Gewinde, sodass zunächst die Schraube durch ein erstes Knochenfragment in ein zweites Knochenfragment geschraubt wird und dann auf dem metrischen Gewinde eine Mutter angesetzt wird, mit der ein Anpressdruck auf das erste Knochenfragment in Richtung zum zweiten Knochenfragment erzeugt wird. Des Weiteren sind Schrauben bekannt, die an einem Ende ein Knochengewinde mit einer ersten definierten Steigung und am anderen Ende ein Knochengewinde mit einer zweiten definierten Steigung aufweisen, wobei die zweite Steigung größerer oder kleinerer als die erste Steigung ist, so dass der Anpressdruck beim Einschrauben durch die unterschiedlichen Vorschübe der Knochenfragmente bewirkt wird. Ebenso ist bekannt, bei Implantaten anstelle eines Knochengewindes einen anderen Verankerungsmechanismus in einem Knochen vorzusehen, beispielsweise kann dies durch die keilförmige Ausgestaltung eines Implantats erreicht werden, wobei das Implantat dann mit der Spitze zuerst, zumeist in einen vorgebohrten Kanal, in einen Knochen eingeführt wird und durch Aufbringen einer Kraft auf das Implantat mit dem Knochen verklemmt wird.

Die bekannten Implantate zur Stabilisierung von Beckenringfrakturen ermöglichen es, Knochenfragmente gegeneinander zu pressen und so den Heilungsvorgang zu unterstützen. Allerdings weisen die bekannten Implantate den Nachteil auf, dass durch sie keine winkelstabile Fixierung von Knochenfragmenten, insbesondere keine winkelstabile transsakrale und/oder transiliale Fixierung erzeugt werden kann. Für eine winkelstabile Fixierung, also eine Fixierung, durch die ein Verdrehen der Knochenfragmente gegeneinander zumindest teilweise vermieden wird, ist vielmehr das Vorsehen mehrerer der bekannten Implantate notwendig. Beispielsweise können die Implantate in verschiedenen Winkeln entlang der Bruchebene zwischen zwei Knochenfragmenten in die Knochenfragmente eingebracht werden. Dies bringt jedoch den Nachteil mit sich, dass durch die vielfachen Bohrungen und/oder Verschraubungen und/oder Verklemmungen die miteinander zu fixierenden Knochenfragmente, insbesondere in der Nähe der Fraktur, geschwächt werden. Außerdem bringen die beschriebenen bekannten Implantate den Nachteil mit sich, dass der Anpressdruck zwischen den Knochenfragmenten durch das Implantat selbst erzeugt wird, wodurch die Kontaktstellen zwischen Implantat und Knochen belastet werden, was zu Schädigungen am Knochen, insbesondere am Übergang zwischen Implantat und Knochen, der für die Halterung des Knochens besonders relevant ist, führen

Ein Implantat mit den Merkmalen des Oberbegriffs des Anspruchs 1 ist aus der US 5 108 397 A bekannt. Ausgehend von dem Stand der Technik liegt der Erfindung die Aufgabe zugrunde, ein Implantat zur Stabilisierung von Beckenringfrakturen bereitzustellen, durch das die beschriebenen Probleme bei herkömmlichen Implantaten zumindest teilweise behoben werden. In gleicher Weise soll die Erfindung möglicherweise auch zur Durchführung einer Arthrodese bei degenerativen Instabilitäten und/oder Arthrosen des Sakroilialgelenks verwendet werden können.

Als eine Lösung der genannten technischen Aufgabe schlägt die Erfindung ein Implantat mit den Merkmalen von Anspruch 1 vor. Das entsprechende erfindungsgemäße Implantat umfasst einen Nagel zur intraossären Implantation sowie zumindest ein Verriegelungselement zur relativen Fixierung von Beckenknochen zum Nagel. Zudem umfasst das erfindungsgemäße Implantat ein Fixierungsmittel. Intraossäre Implantation bezeichnet dabei eine Implantation innerhalb eines Knochens. Entsprechend ist der Nagel des erfindungsgemäßen Implantats so ausgestaltet, dass der Nagel durch einen durch ein Sacrum vorgebohrten Durchführungskanal durch das Sacrum intraossär durchführbar oder durch einen in dem Sacrum vorgebohrten Durchführungskanal intraossär in das Sacrum einführbar ist. Der Nagel weist einen Durchmesser von 7mm bis 10 mm und eine Länge von 80 mm bis 220 mm auf. Der Nagel weist zumindest an seinem ersten Nagelende eine transversale Durchführung zur Aufnahme des Verriegelungselements auf. Das Verriegelungselement weist einen Verankerungsabschnitt auf, mittels dessen es in einem Beckenknochen verankerbar ist. Zudem weist das Verriegelungselement einen Verriegelungsabschnitt auf, der mit der transversalen Durchführung des Nagels so korrespondiert, dass das Verrieglungselement winkelstabil mit dem Nagel verriegelbar ist. Das erfindungsgemäße Implantat zeichnet sich dadurch aus, dass der Nagel so ausgestaltet ist, dass er durch einen durch den S1- oder den S2-Korridor des Sacrums vorgebohrten Durchführungskanal intraossär durchführbar oder in einen in den S1- oder S2-Korridor des Sacrums vorgebohrten Durchführungskanal intraossär einführbar ist, ohne eine Fixierung der Beckenringfraktur in Nagellängsrichtung zu gewährleisten, und dann an seinem zweiten Nagelende mittels des Fixierungsmittels in einem ersten Beckenknochen und/oder in dem Sacrum fixierbar ist und dann eine Kraft in Nagellängsrichtung zur lateralen Druckbeaufschlagung von außen auf den Beckenring unter Kompression des Beckenrings im Bereich der Längserstreckung des Nagels ausübbar ist. Weiterhin ist der Nagel mittels des Verriegelungselements relativ zu einem zweiten Beckenknochen gegen Verdrehungen und Verschiebungen gesichert fixierbar unter winkelstabiler Aufrechterhaltung der Kraft auf den Beckenring im Bereich der Längserstreckung des Nagels. Somit kann der Nagel, nachdem er an seinem zweiten Nagelende in einem ersten Beckenknochen und/oder in dem Sacrum mittels eines Fixierungsmittels fixiert worden ist, mittels des Verriegelungselements relativ zu einem zweiten Beckenknochen gegen Verdrehungen und Verschiebungen gesichert fixierbar sein.

Der Nagel kann somit zunächst mittels des Fixierungsmittels an seinem zweiten Nagelende in einem Beckenknocken und/oder einem Sacrum fixiert werden und sodann mittels des Verriegelungselementes winkelstabil verankert werden.

Beispielsweise kann der Nagel unilateral eingesetzt werden. Dabei kann der Nagel beispielsweise zur Stabilisierung einer Sacrumfraktur durch einen Durchführungskanal in das Sacrum eingeführt und mittels des Fixierungsmittels in dem Sacrum fixiert werden, wonach die winkelstabile Verankerung in einem Beckenknochen, beispielsweise in einem Ilium, erfolgen kann. Damit ist die Fraktur in dem Sacrum durch das erfolgte Fixieren des Sacrums gegenüber dem weiteren Beckenknochen fixiert. Die Fraktur liegt dann vorteilhafterweise zwischen dem Fixierungspunkt des Nagels in dem Sacrum, der durch das Zusammenwirken zwischen Sacrum und Fixierungsmittel festgelegt wird, und dem Fixierungspunkt des Nagels in einem Beckenknochen, der durch das Zusammenwirken des Verriegelungselements mit dem Beckenknochen festgelegt wird. Gleichwohl kann der Nagel auch bilateral eingesetzt werden, beispielsweise durch einen Durchführungskanal durch das Sacrum geführt werden und an seinen beiden Enden zumindest abschnittsweise außerhalb des Sacrums, beispielsweise jeweils in einem Ilium, fixiert werden, zunächst an seinem zweiten Ende mittels des Fixierungsmittels, sodann an seinem ersten Ende mittels des Verriegelungselements.

Der Verankerungsabschnitt des Verriegelungselements ist so ausgebildet, dass er in einem Beckenknochen verankerbar ist. Beispielsweise kann der Verankerungsabschnitt keilförmig ausgebildet sein und/oder ein Knochengewinde umfassen. Auch kann der Verankerungsabschnitt eine Oberfläche aufweisen, die leicht mit einem Beckenknochen verwachsen kann. Der Verriegelungsabschnitt des Verriegelungselements ist über die transversale Durchführung des Nagels mit dem Nagel verriegelbar. Beispielsweise kann der Verriegelungsabschnitt so ausgebildet sein, dass der Verriegelungsabschnitt mit der transversalen Durchführung des Nagels verklemmbar ist. Beispielsweise kann die Oberfläche des Verriegelungsabschnitts Rasthaken aufweisen, oder der Verriegelungsabschnitt kann keilförmig ausgebildet sein. Auch ist eine Ausgestaltung des Verriegelungsabschnitts nach Art eines Spaltdübels möglich. Weiterhin kann der Verriegelungsabschnitt ein Gewinde aufweisen, das mit einem in der transversalen Durchführung des Nagels vorgesehenen Gewinde korrespondiert. Sodann kann die Verriegelung zwischen Verriegelungselement und Nagel dadurch erfolgen, dass das Verriegelungselement mit dem Nagel verschraubt wird. Der Verriegelungsabschnitt und der Verankerungsabschnitt des Verriegelungselements können beispielsweise direkt aneinander liegen, nahtlos ineinander übergehen oder aber auch voneinander beabstandet sein.

Die Aufnahme des Verriegelungselements in dem Nagel kann dadurch erfolgen, dass das Verriegelungselement mit seinem ersten Ende zuerst durch die transversale Durchführung des Nagel durchgeführt wird. Es kann vorteilhaft sein, dass das Verriegelungselement an seinem ersten Ende konisch abgeflacht ist. Dadurch kann das Verriegelungselement leichter in die transversale Durchführung des Nagels eingeführt werden; insbesondere kann das Verriegelungselement dann auch bei einer Verkippung des Verriegelungselements gegenüber der Erstreckungsrichtung der transversalen Durchführung ohne größere Probleme in die transversale Durchführung eingeführt werden. Die konische Abflachung des Verriegelungselements an seinem ersten Ende ist auch gerade dann vorteilhaft, wenn das Verriegelungselement unter Aufbringung einer erheblichen Kraft in die transversale Durchführung eingeführt wird. Vorteilhafterweise ist an einem Ende des Verriegelungselements ein Werkzeuganschluss vorgesehen. Der Werkzeuganschluss kann beispielsweise als Sechskant oder nach Art eines sonstigen Schraubenkopfs ausgebildet sein, damit ein Schrauben des Verriegelungselements problemlos möglich ist. Beispielsweise kann als Werkzeuganschluss auch eine sonstige Koppelungsvorrichtung vorgesehen sein, die mit einem Werkzeug koppelbar ist.

Der Nagel, der in dem erfindungsgemäßen Implantat zum Einsatz kommt, weist einen solchen Durchmesser auf, dass er durch einen durch das Sacrum vorgebohrten Durchführungskanal intraossär durchführbar ist, und dass gleichzeitig in dem Nagel transversale Durchführungen zur Aufnahme eines Verriegelungselements vorgesehen werden können. Entsprechend ist eine gewisse Mindestdicke des Nagels erforderlich, doch darf der Nagel nicht eine solche Dicke erreichen, dass er durch das Sacrum nicht mehr durchführbar ist, beispielsweise da ansonsten eine erhebliche Schwächung des Sacrums oder ein Kontakt mit den am Sacrum verlaufenden Nerven zu befürchten ist.

Vorzugsweise kann der Nagel in dem erfindungsgemäßen Implantat eine solche Länge aufweisen, dass der Nagel nach dem Durchführen durch den Durchführungskanal in dem Sacrum an seinem zweiten Nagelende in einem ersten Beckenknochen, insbesondere in einem ersten Ilium, mittels eines Fixierungsmittels fixierbar ist und nach der Fixierung der Nagel an seinem ersten Ende mittels des Verriegelungselements, das in seiner transversalen Durchführung verankerbar ist, in einem zweiten Beckenknochen, insbesondere in dem zweiten Ilium verankerbar ist. Der Nagel muss in dieser Ausführungsform daher länger sein als die Länge des Durchführungskanals durch das Sacrum. Die Länge des Durchführungskanals hängt dabei zum einen von der individuellen Breite des Sacrums ab, durch das der Nagel durchzuführen ist, zum anderen von der Stelle an dem Sacrum, an der der Durchführungskanal vorgesehen ist. Das Sacrum wird im menschlichen Körper von oben nach unten hin schmaler und weist Bereiche auf, in denen es über seine gesamte Breite als durchgehender Knochen ausgebildet ist. Diese Bereiche werden als S1-, S2, S3-, S4 oder S5-Korridor bezeichnet. Die Bereiche entsprechen jeweils einem Wirbel, wobei in dem Sacrum die 5 Wirbel, die die genannten Korridore bilden, miteinander verwachsen sind. Der S1-Korridor ist der Korridor in dem Sacrum, der im menschlichen Körper am höchsten gelegen ist und an dessen Stelle das Sacrum am breitesten ist. Die Breite des Sacrums nimmt nach unten hin, also vom S1-Korridor zum S5-Korridor hin ab. Entsprechend ist, je nachdem, in welchem Korridor des Sacrums der Durchführungskanal vorgesehen ist, durch den der Nagel des erfindungsgemäßen Implantats durchgeführt werden soll, eine entsprechende Länge des Nagels vorzusehen.

Vorzugsweise kann der Nagel in dem erfindungsgemäßen Implantat eine solche Länge aufweisen, dass der Nagel nach dem Einführen durch den Durchführungskanal in das Sacrum an seinem zweiten Nagelende in dem Sacrum mittels eines Fixierungsmittels fixierbar ist und nach der Fixierung der Nagel an seinem ersten Ende mittels des Verriegelungselements, das in seiner transversalen Durchführung verankerbar ist, in einem Beckenknochen, insbesondere in einem Ilium verankerbar ist. In dieser Ausführungsform muss der Nagel eine solche Länge aufweisen, dass er sich nach dem Einführen und Fixieren in dem Sacrum mit seinem ersten Ende außerhalb des Sacrums über einen Iliumbereich erstreckt, jedoch ist die Länge des Nagels so zu beschränken, dass der Nagel keine Schädigungen außerhalb des Beckenrings hervorrufen kann.

Vor der Fixierung des Nagels relativ zu einem zweiten Beckenknochen mittels des Verriegelungselements ist zunächst eine Fixierung des Nagels an seinem zweiten Nagelende in einem ersten Beckenknochen und/oder in einem Sacrum mittels eines Fixierungsmittels erforderlich. Das Fixierungsmittel kann verschiedenartig ausgestaltet sein, insbesondere umfasst das Fixierungsmittel in einer Ausführungsform kein Verriegelungselement. Beispielsweise kann als Fixierungsmittel ein Gewinde an dem zweiten Nagelende vorgesehen sein, insbesondere nach Art eines Knochengewindes, sodass der Nagel in den ersten Beckenknochen und/oder in das Sacrum schraubbar ist. Auch kann als Fixierungsmittel eine keilförmige Ausgestaltung des Nagels vorgesehen sein, sodass der Nagel in den ersten Beckenknochen und/oder das Sacrum einschlagbar ist. Dem Fachmann sind verschiedene Möglichkeiten bekannt, Fixierungsmittel zur Fixierung eines Nagels in einem Knochen vorzusehen, derer er sich bei der Realisierung des Fixierungsmittels zur Fixierung des Nagels an seinem zweiten Ende in dem ersten Beckenknochen und/oder dem Sacrum bedienen kann. Das Fixierungsmittel kann insbesondere so ausgebildet sein, dass der Nagel mittels des Fixierungsmittels in einem Knochen so fixierbar ist, dass er dem Knochen gegenüber bei Aufbringen einer Kraft auf den Nagel in Richtung der axialen Erstreckung des Nagels gegen eine Verschiebung gesichert ist. Diese Kraft ist insbesondere eine solche, welche zur Anordnung und lateralen Druckbeaufschlagung der anatomischen Teile des Beckens wie z.B. Illium und/oder Sacrum in ihrer Sollposition, welche zur Heilung des Bruches oder Fixierung der Beckenringanatomie zu erreichen bzw. aufzubringen ist, durch das Implantat in Nagellängsrichtung auf den Beckenring ausgeübt wird. Insbesondere ist es auch möglich, als Fixierungsmittel ein zweites Verriegelungselement vorzusehen, sowie an dem Nagel eine zweite transversale Durchführung vorzusehen, sodass der Nagel zuerst an seinem zweiten Ende mit dem Verriegelungselement verankerbar ist und sodann mit einem weiteren Verriegelungselement relativ zu einem Beckenknochen gegen Verdrehungen und Verschiebungen gesichert fixierbar ist. Selbstverständlich können zur Fixierung des Knochens an einem seiner Enden auch mehrere diesem einen Ende zugeordnete Verriegelungselemente und mehrere transversale Durchführungen an diesem einen Ende vorgesehen sein.

Ein bedeutender Vorteil der Erfindung liegt darin, dass bei der Implantation des erfindungsgemäßen Implantats zunächst eine Fixierung des Nagels einem ersten Beckenknochen möglich ist und dann von außen auf den Beckenring eine Kraft ausgeübt werden kann, wodurch die Knochen entlang der Bruchstelle aneinander gepresst werden, wonach dann durch die Verankerung des Verriegelungselements in dem zweiten Beckenknochen der Nagel relativ zu dem zweiten Beckenknochen gegen Verdrehungen und Verschiebungen gesichert fixierbar ist. Somit muss der Druck auf die Bruchstelle in dem gebrochenen Knochen nicht durch das Implantat selbst bei dem Einbringen des Implantats erzeugt werden, sondern der Druck kann von außen erzeugt werden, und das Verriegelungselement kann den Nagel lediglich in der von außen eingestellten Position verankern. Dieser Vorteil tritt auch bei dem unilateralen Einsatz des erfindungsgemäßen Implantats auf, wenn der Nagel zuerst an seinem zweiten Ende in einem Sacrum fixiert und sodann an seinem ersten Ende in einem Beckenknochen verankert wird, nachdem eine Kompression von außen auf den Beckenring ausgeübt wurde, in den das Implantat implantiert wird.

Die Implantation eines erfindungsgemäßen Implantats kann beispielsweise mit einem Zielgerät erfolgen, das an das erste Ende des Nagels ankoppelbar ist. Der Nagel kann möglicherweise mit Hilfe des Zielgeräts in den Durchführungskanal eingeführt oder durch ihn durchgeführt werden, wobei das Zielgerät so auf den Nagel des Implantats abgestimmt ist, dass das Zielgerät dem Operateur vorgibt, an welcher Stelle und in welcher Bohrrichtung das zumindest eine Verriegelungselement in den menschlichen Körper zur Gewährleistung der Verankerung des Nagels in einem Beckenknochen einzubringen ist. Das Zielgerät kann insbesondere während des Aufbringens des Kompressionsdrucks von außen auf den Beckenring mit dem Nagel verbunden sein.

Ein weiterer bedeutender Vorteil der Erfindung liegt darin, dass das Verriegelungselement so in die transversale Durchführung des Nagels einbringbar ist, dass das Verriegelungselement winkelstabil mit dem Nagel verriegelbar ist. Durch die Verankerung des Verriegelungselements in dem Beckenknochen ist dann der Nagel winkelstabil gegenüber dem Beckenknochen angeordnet. Dadurch kann effektiv einem Verdrehen von Knochenfragmenten gegeneinander vorgebeugt werden.

Insbesondere ist bei dem erfindungsgemäßen Implantat kein mehrfaches Durchbohren und/oder Durchschlagen der Bruchstelle zur Fixierung der Bruchstelle erforderlich. Vielmehr ist lediglich eine einzige Durchbohrung des Sacrums zur Erzeugung des Durchführungskanals notwendig, durch den dann der Nagel ein- oder durchgeführt wird, wonach der Nagel an seinen beiden Enden jeweils in einem oder mehreren Beckenknochen verankerbar ist. Somit eignet sich das erfindungsgemäße Implantat insbesondere zur Fixierung eines Bruchs im Iliosakralgelenk oder im Sacrum. Für die Fixierung eines solchen Bruchs ist es besonders vorteilhaft, dass die Verankerung des Verriegelungselements - nach der Fixierung des Nagels an seinem zweiten Nagelende mittels des Fixierungsmittels - zumindest teilweise in dem Beckenknochen und nicht in dem Sacrum oder dem Iliosakralgelenk erfolgen kann, sodass keine Beschädigung der Bruchstelle zu erfolgen hat. Vielmehr kann die Bruchstelle durch eine einfache und sauber ausführbare Durchbohrung durchbohrt werden, wobei die Kompression der zu verbindenden Knochenelemente nicht durch das Implantat, sondern von außen, beispielsweise durch eine externe an das Implantat adaptierbare Zwinge und/oder ein Zielgerät, ausgeübt werden kann und die so fixierte Position der Knochenelemente zueinander durch die Verankerung des Verriegelungselements außerhalb des geschädigten Knochens erfolgen kann. Das erfindungsgemäße Implantat ist insbesondere so ausgebildet, dass durch die Verankerung des Implantats mittels des Verriegelungelements an seinem ersten Nagelende nach erfolgter Fixierung des Nagels an seinem zweiten Nagelende keine Kompression der zu verbindenden Knochenelemente erfolgt. Das erfindungsgemäße Implantat ist insbesondere so ausgebildet, dass durch die Verankerung mittels des Verriegelungselements eine von außen auf die zu fixierenden Beckenknochen aufgebrachte Kompression, die nach der Fixierung des Nagels an seinem zweiten Nagelende erfolgt, winkelstabil aufrechterhalten ist.

Die Ein- oder Durchführung des Nagels durch den Durchführungskanal in oder durch das Sacrum hindurch kann beispielsweise dadurch erfolgen, dass ein kanülierter Nagel verwendet wird, wobei der Durchführungskanal über einen Führungsdraht gebohrt wird und der Nagel danach über den Führungsdraht durch den Durchführungskanal durchgeführt wird, wobei der Führungsdraht durch die Kanülierung in dem Nagel verläuft.

Erfindungsgemäß ist der Nagel so ausgestaltet, dass der Nagel durch einen durch den S1- oder den S2-Korridor eines Sacrums vorgebohrten Durchführungskanal intraossär durchführbar ist, wobei der Nagel einen Durchmesser von 7 mm bis 10 mm und eine Länge von 80 mm bis 220 mm aufweist. Der durch den entsprechenden Korridor vorgebohrte Durchführungskanal kann insbesondere einen kleineren Durchmesser als der entsprechende Korridor aufweisen, um Schädigungen von Nerven außerhalb des Korridors und/oder eine Schwächung des Sacrums zu vermeiden. Der S1-Korridor ist der Korridor in dem Sacrum, der am breitesten und am höchsten ist und der am meisten Knochensubstanz bietet, sodass gerade in dem S1-Korridor eine Durchbohrung des Sacrums und eine anschließende Belastung des Sacrums möglich ist, ohne dass es zu Beschädigungen des Sacrums kommt. Der S2-Korridor ist zwar kleiner als der S1-Korridor und weist weniger Knochensubstanz als der S1-Korridor, aber ist größer als der S3-Korridor, der S4-Korridor und der S5-Korridor. Insgesamt genügt die vom S2-Korridor bereitgestellte Knochensubstanz in den meisten Fällen immer noch für die Durchbohrung und anschließende Implantation eines Nagels, sodass auch in dem S2-Korridor die Implantierung des Nagels ohne Schädigung des Sacrums möglich sein kann. Durch das Vorsehen eines Nageldurchmessers von 7 mm bis 10 mm kann insbesondere ein Gleichgewicht gewahrt sein zwischen einer möglichst belastbaren Ausgestaltung des Nagels und einer möglichst geringen Belastung des Sacrums, indem der Durchmesser des Durchführungskanals klein gehalten wird. Das Vorsehen einer Länge von 80 mm bis 220 mm beim Nagel kann ermöglichen, dass der Nagel in einem Großteil der Beckenringe erwachsener Menschen so durch das Sacrum anordenbar ist, dass die Nagelenden jeweils ungefähr in der Mitte der das Sacrum umschließenden Ilia liegen, wobei als "Mitte" des Iliums die Mitte in Bezug auf die seitliche Erstreckung des Iliums bei Betrachtung des Beckenrings von vorne bezeichnet ist. Bei einem solchen bilateralen Einsatz des erfindungsgemäßen Implantats kann insbesondere das Vorsehen eine Nagellänge von 140 mm bis 180 mm vorteilhaft sein.

Ferner ist aber auch gewährleistet, dass das erfindungsgemäße Implantat mit einer kürzeren Nagellänge unilateral eingesetzt werden kann, indem der Nagel nicht durch-, sondern nur in das Sacrum eingeführt wird und die Fixierung des Nagels an seinem zweiten Nagelende nicht im Ilium sondern im Sacrum erfolgt. Hierzu kann insbesondere das Vorsehen eine Nagellänge von 80 mm bis 140 mm vorteilhaft sein

Vorteilhafterweise weist die transversale Durchführung ein Innengewinde auf, das mit einem an dem Verriegelungsabschnitt des Verriegelungselements vorgesehenen Gewinde korrespondiert. Insbesondere kann damit das Verriegelungselement mit dem Nagel verschraubbar sein. Dem Fachmann ist klar, dass hierzu jede Gewindeart, über die eine Verschraubung zweier Elemente möglich ist, geeignet sein kann. Insbesondere kann das Innengewinde der transversalen Durchführung nach Art eines Knochengewindes ausgestaltet sein, das mit einem an dem Verriegelungsabschnitt des Verriegelungselements angeordneten Knochengewinde korrespondiert. Insbesondere kann es auch vorteilhaft sein, als Innengewinde in der transversalen Durchführung und als Außengewinde an dem Verriegelungsabschnitt ein metrisches Gewinde vorzusehen. Durch das metrische Gewinde kann ein Anzugsmoment möglicherweise besonders gut definiert werden, wobei die Steigung des Gewindes zum Erzielen einer möglichst sicheren Verriegelung zwischen Nagel und Verriegelungselement festlegbar ist.

Insbesondere kann zumindest an dem Verankerungsabschnitt des Verriegelungselements ein Knochenschraubengewinde vorgesehen sein. Durch das Knochenschraubengewinde kann durch einfaches Einschrauben des Verriegelungselements in einen Beckenknochen eine Verankerung des Verriegelungselements, und damit auch des Nagels, an dem Beckenknochen erzielt werden. Beispielsweise kann das Knochengewinde ununterbrochen von dem Verankerungsabschnitt in den Verriegelungsabschnitt übergehen. Zwischen Verankerungsabschnitt und Verriegelungsabschnitt kann jedoch beispielsweise auch ein Gewindewechsel erfolgen, beispielsweise ein Wechsel der Art des Gewindes oder ein Wechsel der Steigung des Knochenschraubengewindes. Selbstverständlich kann der Verankerungsabschnitt ein Knochenschraubengewinde aufweisen und der Verriegelungsabschnitt nicht als Gewinde sondern anderweitig zur Herstellung der Verriegelung ausgestaltet sein. Das Vorsehen von Gewinden sowohl am Verankerungsabschnitt als auch am Verriegelungsabschnitt kann jedoch den Vorteil mit sich bringen, dass das Verriegelungselement während des Einführens in die transversale Durchführung mittels Drehens und Belastens in Durchführungsrichtung gleichzeitig mit dem Nagel verriegelbar und in einem Knochen verankerbar ist.

Vorteilhafterweise kann der Durchmesser des Verriegelungselements in dem Verriegelungsabschnitt größer sein als in dem Verankerungsabschnitt. Dies bringt den Vorteil mit sich, dass der Verankerungsabschnitt zuerst durch die transversale Durchführung durchgeführt werden kann, ohne dass es zu einer Schädigung der transversale Durchführung kommt, da entsprechend dem größeren Durchmesser am Verriegelungsabschnitt auch der Durchmesser der transversalen Durchführung größer sein muss als der in dem Verankerungsabschnitt.

Weiterhin kann dadurch der Verriegelungsmechanismus zwischen Verriegelungsabschnitt und transversaler Durchführung vollkommen unabhängig von dem Verankerungsmechanismus zwischen Knochen und Verankerungsabschnitt ausgebildet sein.

Weiterhin kann der erste Verankerungsabschnitt an einem ersten Ende des Verriegelungselements angeordnet sein, wobei der Verriegelungsabschnitt von dem ersten Ende des Verriegelungselements aus gesehen hinter dem Verankerungsabschnitt angeordnet ist, wobei entsprechend gesehen hinter dem Verriegelungsabschnitt ein Abschnitt des Verriegelungselements angeordnet ist, der zapfenartig ausgebildet ist und dessen Durchmesser zumindest genauso groß ist wie der Außendurchmesser des Verriegelungselements im Verriegelungsabschnitt.

Dadurch ist es möglich, das Verriegelungselement ohne Beschädigung des Innenbereichs der transversalen Durchführung durch die transversale Durchführung mit einem ersten Ende durchzuführen und danach das Verriegelungselement mit seinem Verriegelungsabschnitt in der transversalen Durchführung zu verriegeln. Dadurch, dass der Durchmesser des Zapfenabschnitts zumindest genauso groß ist wie der Außendurchmesser des Verriegelungsabschnitts, wobei der Zapfenabschnitt vom ersten Ende aus gesehen hinter dem Verriegelungsabschnitt angeordnet ist, kann der Zapfenabschnitt so ausgestaltet sein, dass er sehr genau in die transversale Durchführung eingepasst ist. Bei einer solchen genauen Einpassung des Zapfens in der transversalen Durchführung und einer gewissen Längenerstreckung des Zapfenabschnitts ist nach dem Verriegeln des Verriegelungselements über den Verriegelungsabschnitt mit dem Nagel eine besonders gute winkelstabile Fixierung zwischen Verriegelungselement und Nagel realisierbar. Beispielsweise kann der Verriegelungsabschnitt ein Gewinde aufweisen und der Zapfen beim Einschrauben des Verriegelungselements in ein Innengewinde in der transversalen Durchführung mit seinem dem ersten Ende zugewandten Ende an das Gewinde und/oder einen Vorsprung anstoßen, sodass bei dem Festschrauben des Verriegelungselements an dem Nagel der Zapfen gegen das entsprechende Element (Gewinde/Vorsprung) des Nagels gepresst wird. Bei dem Vorsehen eines entsprechenden Gewindes mit einer entsprechenden Steigung sowie einem entsprechenden Anzugsdrehmoment kann eine besonders gute Verbindung zwischen Verriegelungselement und Nagel gewährleistet sein, beispielsweise kann ein Kaltverschweißen zwischen Verriegelungselement und Nagel realisierbar sein.

Weiterhin kann zumindest eines der Verriegelungselemente einen zweiten Verankerungsabschnitt aufweisen, wobei der Verriegelungsabschnitt zwischen dem ersten und dem zweiten Verankerungsabschnitt angeordnet ist, und wobei der zweite Verankerungsabschnitt nach Art eines Knochengewindes ausgestaltet ist. Bei dieser Ausgestaltung ist es möglich, den Nagel mit dem Verriegelungselement über den Verriegelungsabschnitt zu verriegeln und eine Verankerung des Verriegelungselements sowohl oberhalb als auch unterhalb des Nagels in einem Knochen zu realisieren. Beispielsweise können der erste und der zweite Verankerungsabschnitt in einem Ilium verankert werden. Es ist jedoch auch möglich, bei der Implantation des Implantats zumindest einen der beiden Verankerungsabschnitte zumindest teilweise in dem Sacrum oder einem anderen Knochen des Beckenrings zu verankern. Somit kann durch das Vorsehen von erstem und zweitem Verankerungsabschnitt eine besonders gute Verankerung des Verriegelungselements ober- und unterhalb des Nagels gewährleistet werden, insbesondere in mehreren, verschiedenen Beckenknochen.

Vorteilhafterweise ist der Durchmesser des Verriegelungselements in dem zweiten Verankerungsabschnitt größer als der Durchmesser des Verriegelungselements in dem ersten Verankerungsabschnitt. Da der zweite Verankerungsabschnitt zur Verrieglung des Verriegelungselements mit dem Nagel nicht durch die transversale Durchführung im Nagel durchgeführt werden muss, bewirkt das Vorsehen eines größeren Durchmessers in dem zweiten Verankerungsabschnitt auch keine Schädigung der transversalen Durchführung in dem Nagel. Der größere Durchmesser kann jedoch eine besonders stabile Verankerung in einem Knochen ermöglichen.

Vorteilhafterweise kann der Nagel an beiden Enden antitraumatisierend ausgebildet sein. Dies ist beispielsweise durch das Vorsehen von Kappen an jedem Nagelende möglich. Durch die entsprechende Ausbildung des Nagels können die Nagelanschlüsse funktional bleiben, und eine Reizung des den Nagel umgebenden Gewebes durch die Nagelenden kann möglicherweise vermieden werden.

Vorteilhafterweise kann das Implantat zumindest zwei Verriegelungselemente aufweisen, wobei der Nagel zumindest zwei transversale Durchführungen zur Aufnahme jeweils eines der Verriegelungselemente aufweist, wobei das Fixierungsmittel zur Fixierung des Nagels an seinem zweiten Nagelende eines der zumindest zwei Verriegelungselemente umfasst, wobei zwischen zumindest zwei der transversalen Durchführungen ein Abstand vorgesehen ist, der mindestens 70 % der Länge des Nagels beträgt. Nach dem Durchführen des Nagels können die Verriegelungselemente jeweils außerhalb des Sacrums in einem Iliumknochen verankerbar sein. Die Verriegelungselemente können, müssen aber nicht zwingend jeweils identisch ausgestaltet sein. Allerdings ist jedes der Verriegelungselemente so ausgestaltet, dass es mit zumindest einer der transversalen Durchführungen im Nagel korrespondiert. Dadurch, dass das Fixierungsmittel eines der Verriegelungselemente umfasst, kann der Nagel auch an seinem zweiten Ende winkelstabil mit einem Beckenknochen verankert werden. Darüber hinaus kann das Fixierungsmittel weitere Mittel umfassen, wie beispielsweise eine keilfömige Ausgestaltung des Nagels an seinem zweiten Nagelende oder ein weiteres Verriegelungselement, das beispielsweise mit dem ersten vom Fixierungsmittel umfassten Verriegelungselement oder mit einer weiteren transversalen Durchführung korrespondiert. In einer vorteilhaften Ausführungsform umfasst das Implantat genau zwei Verriegelungselemente sowie genau zwei transversale Durchführungen in dem Nagel, wobei die transversalen Durchführungen jeweils nahe einem Ende des Nagels angeordnet sind. Durch die Ausgestaltung von Verriegelungselement und Nagel kann bei dem entsprechenden Vorsehen zweier Verriegelungselemente sowie transversaler Durchführungen an jedem der Nagelenden auf sehr einfache Weise an jedem der Nagelenden eine winkelstabile Fixierung des Nagels in einem Beckenknochen erreicht werden. Dadurch können möglicherweise die Beckenknochen selbst gegeneinander winkelstabil fixiert sein. Die winkelstabile Fixierung der Beckenknochen gegeneinander kann möglicherweise auch eine winkelstabile Fixierung von Sacrumfrakturen entlang einer Bruchstelle in dem Sacrum gewährleisten, indem vor der Verankerung des Verriegelungselements an dem ersten Nagelende die das Sacrum umgebenden Beckenknochen gegeneinander gedrückt werden, sodass Druck auf die Bruchstelle in dem Sacrum ausgeübt wird, sodass die Sacrumfragmente entlang der der Bruchstelle ineinandergreifen, sodass bei einer winkelstabilen, druckbelasteten Verriegelung des Nagels über die Verriegelungselemente in den Beckenknochen auch eine winkelstabile Fixierung von Sacrumfragmenten realisierbar ist.

Die in dem letzten Absatz beschriebenen Vorteile und Ausgestaltungen sind auf entsprechende Art und Weise auch auf ein erfindungsgemäßen Implantat anwendbar, wenn zumindest zwei Verriegelungselemente vorgesehen sind, wobei der Nagel zumindest zwei transversale Durchführungen zur Aufnahme jeweils eines der Verriegelungselemente aufweist, wobei das Fixierungsmittel zur Fixierung des Nagels an seinem zweiten Nagelende eines der zumindest zwei Verriegelungselemente umfasst, wobei zwischen zumindest zwei der transversalen Durchführungen ein solcher Abstand vorgesehen ist, dass nach dem Einführen des Nagels in das Sacrum eines der Verriegelungselemente in dem Sacrum und eines der Verrieglungselemente außerhalb des Sacrums in einem Iliumknochen verankerbar sind. Die Vorteile ergeben sich dann in Bezug auf die Stabilisierung von Knochenfragmenten beim unilateralen Einsatz des Nagels.

Allgemein ist festzuhalten, dass sowohl an dem ersten Nagelende als auch an dem zweiten Nagelende als auch an weiteren Stellen des Nagels jeweils ein oder mehrere transversale Durchführungen angeordnet sein können, denen jeweils ein Verriegelungselement zugeordnet ist, um eine möglichst gute Fixierung des Implantats und damit der zu stabilisierenden Knochenfragmente zu erreichen.

Weiterhin können zumindest eine oder sämtliche der transversalen Durchführungen eine Erstreckungsrichtung durch den Nagel aufweisen, die einen Winkel von größer 0°, vorzugsweise zwischen 5° und 60°, zur Senkrechten zur Erstreckungsachse des Nagels bildet. Durch ein entsprechendes Verkippen der Erstreckungsrichtung der transversalen Durchführung im Vergleich zur Erstreckungsachse des Nagels kann möglicherweise eine besonders gute Verankerung des Verriegelungselements in dem Knochen gewährleistet sein, insbesondere eine gegenüber Quer- und/oder Längsbelastungen des Nagels stabile Verankerung.

Vorteilhafterweise kann der Nagel zumindest zwei transversale Durchführungen aufweisen, wobei die Erstreckungsrichtung der ersten transversalen Durchführung mit der Erstreckungsrichtung der zweiten transversalen Durchführung in Richtung der Erstreckungsachse des Nagels und/oder in Richtung der Senkrechten zur Erstreckungsachse des Nagels einen Winkel von größer 0° bildet. Durch die Verkippung der transversalen Durchführungen gegeneinander kann zum einen die Implantation des Implantats vereinfacht sein, insbesondere kann dadurch die Verankerung des Verriegelungselements in zumindest einem Beckenknochen und/oder dem Sacrum erleichtert sein. Zum zweiten kann durch die Verkippung eine möglichst stabile Fixierung des Nagels in dem zumindest einen Beckenknochen und/oder dem Sacrum sowie möglicherweise auch eine möglichst stabile Fixierung der Beckenknochen gegeneinander oder eines Beckenknochens zum Sacrum realisierbar sein.

In einer weiteren Ausführungsform bildet die Erstreckungsrichtung der ersten transversalen Durchführung mit der Erstreckungsrichtung der zweiten transversalen Durchführung in Richtung der Erstreckungsachse des Nagels einen Winkel zwischen 5° und 175°, wobei die Durchführungen an der Seite des Nagels, von der aus die Verriegelungselemente in die Durchführungen einführbar sind, weniger weit voneinander entfernt sind als auf der gegenüberliegenden Seite des Nagels. Die Verriegelungselemente bilden in dieser Ausführungsform nach dem Verriegeln mit dem Nagel ein nach oben und unten offenes V, wobei die ersten Enden der Verriegelungselemente, mit denen die Verriegelungselemente durch die transversalen Durchführungen durchgeführt werden, weiter voneinander entfernt sind als die zweiten Enden der Verriegelungselemente. Bei dieser Ausführungsform kann eine besonders gute Verankerung der Verriegelungselemente in zumindest einem Beckenknochen und/oder dem Sacrum realisierbar sein. Darüber hinaus kann eine besonders gute Fixierung der Beckenknochen zueinander und/oder des Sacrums zu zumindest einem Beckenknochen mit dem entsprechenden erfindungsgemäßen Implantat realisierbar sein. Dabei kann es im Sinne einer möglichst guten Stabilisierung von Knochenfragmenten, insbesondere von Sacrumfragmenten, vorteilhaft sein, dass das erfindungsgemäße Implantat zwei Verriegelungselemente umfasst, die jeweils an einem Ende des Nagels angeordnet sind und den Nagel in jeweils einem Ilium verankern, wobei diese beiden Verriegelungselemente gegeneinander ein nach oben und unten offenes V gemäß den obigen Erläuterungen bilden.

Die Erfindung umfasst ferner ein Verriegelungselement zur Verwendung in einem wie oben beschriebenem erfindungsgemäßen Implantat, wobei das Verriegelungselement einen Verankerungsabschnitt und einen Verriegelungsabschnitt aufweist, wobei der Verankerungsabschnitt an einem ersten Ende des Verriegelungselements angeordnet ist und das Verriegelungselement mittels des Verankerungsabschnitts mit einem Beckenknochen und/oder Sacrum verankerbar ist, wobei der Verriegelungsabschnitt von dem ersten Ende des Verriegelungselements aus gesehen hinter dem Verankerungsabschnitt angeordnet ist, wobei der Verriegelungsabschnitt ein Außengewinde aufweist, wobei von dem ersten Ende aus gesehen hinter dem Verriegelungsabschnitt ein Abschnitt angeordnet ist, der zapfenartig ausgebildet ist (Zapfenabschnitt) und dessen Durchmesser zumindest genauso groß ist wie der Außendurchmesser des Verriegelungselements im Verriegelungsabschnitt. Mit Außendurchmesser des Verriegelungselements im Verriegelungsabschnitt ist dabei der absolute Außendurchmesser bezeichnet; bei dem Vorsehen eines Gewindes an dem Verriegelungsabschnitt ist mit Außendurchmesser somit nicht der Schaftdurchmesser, sondern der Außendurchmesser des Gewindes bezeichnet. Durch das Vorsehen des Zapfenabschnitts kann eine besonders gute winkelstabile Fixierung zwischen einem Nagel und dem erfindungsgemäßen Verriegelungselement realisierbar sein.

Das erfindungsgemäße Verriegelungselement kann somit mit seinem ersten Ende durch eine transversale Durchführung eines Nagels durchgeführt werden und mit dem durch den Nagel durchgeführten Verankerungsabschnitt in einem Beckenknochen und/oder Sacrum verankert werden, während der hinter dem Verankerungsabschnitt angeordnete Verriegelungsabschnitt mit dem Nagel verriegelbar ist. Es ist zu berücksichtigen, dass der Verankerungsabschnitt nicht unmittelbar an dem ersten Ende des Verriegelungselements beginnen muss, sondern das erste Ende des Verriegelungselements kann auch auf andere Weise, beispielsweise funktional, ausgestaltet sein, beispielsweise eine konische Abflachung aufweisen, die die Einführung in eine transversale Durchführung erleichtert.

Vorteilhafterweise ist der Durchmesser des Verriegelungselements in dem Verriegelungsabschnitt größer als in dem Verankerungsabschnitt. Dadurch kann der Verankerungsabschnitt durch eine transversale Durchführung durchgeführt werden, ohne dass es zu einer Beschädigung der transversalen Durchführung kommt, während danach der Verankerungsabschnitt über die transversale Durchführung winkelstabil mit dem Nagel fixierbar ist.

Ferner kann das Verriegelungselement einen zweiten Verankerungsabschnitt aufweisen, wobei der Verriegelungsabschnitt zwischen dem ersten und dem zweiten Verankerungsabschnitt angeordnet ist, und wobei der zweite Verankerungsabschnitt nach Art eines Knochengewindes ausgestaltet ist. Durch das Vorsehen von erstem und zweitem Verankerungsabschnitt ist eine besonders gute Fixierung des Verriegelungselements ober- und unterhalb des Verriegelungsabschnitts möglich, wodurch eine besonders gute Verankerung des Nagels in einem Beckenknochen und/oder Sacrum erreichbar ist. Weiterhin kann der Durchmesser des Verriegelungselements in dem zweiten Verankerungsabschnitt größer sein als der Durchmesser des Verriegelungselements in dem ersten Verankerungsabschnitt und/oder in dem Verriegelungsabschnitt. Durch den größeren Durchmesser in dem zweiten Verankerungsabschnitt ist eine besonders gute Fixierung des Verriegelungselements in einem Knochen möglich, ohne dass es bei der Durchführung des Verriegelungselements durch eine transversale Durchführung in dem Nagel zu einer Beschädigung der transversalen Durchführung kommt.

Weiterhin kann das Verriegelungselement ein Verbindungselement an seinem zweiten Ende zur Ankopplung des Verriegelungselements an eine Stabilisierungsmontage der unteren Lendenwirbelkörper aufweisen. Durch das Ankoppeln der Stabilisierungsmontage an das winkelstabil in einem Beckenknochen und/oder Sacrum verankerte Verriegelungselement können die Lendenwirbel besonders stabil mit dem durch das Implantat stabilisierten Beckenring verbunden werden.

Vorteilhafterweise kann das Verriegelungselement axial durchbohrt sein. Demnach kann das Verriegelungselement eine durchgehende Durchbohrung aufweisen, die axial vollständig durch das gesamte Verriegelungselement, nämlich von seinem ersten Ende zu seinem zweiten Ende verläuft. Insbesondere kann die Durchbohrung durch die axiale Mitte des Verriegelungselements verlaufen. Eine entsprechende Durchbohrung wird häufig als Kanülierung bezeichnet. Eine Kanülierung kann insbesondere den Zweck erfüllen, dass das Verriegelungselement zur Implantation eines erfindungsgemäßen Implantats über einen Führungsdraht implantierbar ist. Der Führungsdraht kann in der Kanülierung durch das Verriegelungselement verlaufen, so dass das Verriegelungselement zur Implantation über den Führungsdraht geschoben werden und gezielt implantiert werden kann.

Vorteilhafterweise kann das Verriegelungselement zumindest eine Querbohrung aufweisen. Die Querbohrungen verlaufen zumindest mit einer Richtungskomponente senkrecht zur Achse des Verriegelungselements, wobei die Achse das erste Ende und das zweite Ende des Verriegelungselement verbindet. Die Querbohrungen können beispielsweise als Querdurchbohrungen ausgebildet sein. Die Querbohrungen können jedoch auch als nicht vollständig durch das Verriegelungselement durchgehende Bohrungen vorgesehen sein. Insbesondere kann ein erfindungsgemäßes Implantat sowohl eine axiale Durchbohrung als auch Querbohrungen aufweisen, wobei die Querbohrungen die axiale Durchbohrung kreuzen können oder in die axiale Durchbohrung münden können. Die Querbohrungen können beispielsweise für Knochenstabilisierungsmaßnahmen, insbesondere bei einer Verankerung des Verriegelungselements in Knochen mit schlechter Knochenqualität, vorteilhaft sein. Beispielsweise kann Knochenzement in die Querbohrungen eingebracht werden, der nach und/oder bei dem Verankern des Verriegelungselements aus den Querbohrungen austreten und den Knochen, in dem das Verrieglungselement verankert ist, stabilisieren kann. Beispielsweise kann bei einem Verriegelungselement, in dem eine mit Querbohrungen kommunizierende axiale Durchbohrung vorgesehen ist, Knochenzement über die axiale Durchbohrung in die Querbohrungen eingebracht werden. Das Einbringen des Knochenzements in die Querbohrungen bzw. in die axiale Durchbohrung kann beispielsweise durch Injektion erfolgen.

Die Erfindung umfasst weiterhin einen Nagel zur Verwendung in einem wie oben beschriebenem erfindungsgemäßen Implantat.

In einer Ausführungsform weist der erfindungsgemäße Nagel einen Durchmesser von 7mm bis 10 mm und eine Länge von 80 mm bis 220 mm auf und weist zumindest eine transversale Durchführung an seinem ersten Nagelende auf zur Aufnahme eines korrespondierenden Verriegelungselements, wobei der Nagel so ausgebildet ist, dass er mit seinem zweiten Nagelende durch einen in einen in den S1- oder den S2-Korridor des Sacrums vorgebohrten Durchführungskanal intraossär in das Sacrum einführbar und in dem Sacrum fixierbar oder durch einen durch den S1- oder den S2-Korridor des Sacrums vorgebohrten Durchführungskanal durchführbar und in einem ersten Beckenknochen fixiert ist, ohne eine Fixierung der Beckenringfraktur in Nagellängsrichtung zu gewährleisten, und dann eine Kraft in Nagellängsrichtung zur lateralen Druckbeaufschlagung von außen auf den Beckenring unter Kompression des Beckenrings im Bereich der Längserstreckung des Nagels ausübbar ist, wobei der Nagel mittels des Verriegelungselements relativ zu einem zweiten Beckenknochen gegen Verdrehungen und Verschiebungen gesichert fixierbar ist unter winkelstabiler Aufrechterhaltung der Kraft auf den Beckenring im Bereich der Längserstreckung des Nagels.

Der Nagel weist in einer weiteren Ausführungsform zumindest zwei transversale Durchführungen auf, wobei zwischen den transversalen Durchführungen ein Abstand vorgesehen ist, der mindestens 70 % der Länge des Nagels beträgt. Beispielsweise kann der Nagel durch einen Durchführungskanal in einem Sacrum so durchführbar sein, dass die transversalen Durchführungen nach der Durchführung des Nagels durch den Durchführungskanal jeweils in einem Iliumbereich liegen. Dadurch kann der erfindungsgemäße Nagel in einem Sacrum so implantiert werden, dass er auf jeder Seite des Sacrums an einem Ilium mit einem Verriegelungselement fixierbar ist. Entsprechend ist eine besonders gute, winkelstabile Fixierung des Nagels an beiden Seiten in einem Ilium, und damit der Iliumknochen gegeneinander, möglich. Dies kann insbesondere für die Stabilisierung von Sacrumfrakturen vorteilhaft sein, da dadurch das Sacrum selbst, das zwischen linkem und rechtem menschlichem Ilium angeordnet ist, stabil, insbesondere winkelstabil, fixierbar ist.

In einer weiteren Ausführungsform weist der erfindungsgemäße Nagel zumindest zwei transversale Durchführungen auf, wobei zwischen den transversalen Durchführungen ein solcher Abstand vorgesehen ist, dass der Nagel durch einen Durchführungskanal in ein Sacrum so einführbar ist, dass mindestens eine der transversalen Durchführungen nach der Einführung des Nagels durch den Durchführungskanal in einem Iliumbereich liegt. Entsprechend kann der Nagel eine besonders gute, winkelstabile Fixierung des Sacrums zu einem Iliumknochen ermöglichen, wodurch die zu stabilisierenden Knochenfragmente besonders gut und winkelstabil gegeneinander fixiert werden können.

Vorteilhafterweise weist der Nagel zumindest an einem seiner beiden Enden eine Koppelvorrichtung zum Ankoppeln einer Kompressionszwinge und/oder eines Zielgeräts auf. Dadurch kann der Nagel operativ mit Hilfe eines Zielgeräts in den Durchführungskanal eingeführt werden, was eine einfache und präzise Operationstechnik mit Hilfe des erfindungsgemäßen Nagels erlaubt.

Vorteilhafterweise kann der Nagel axial durchbohrt sein. Demnach kann der Nagel eine durchgehende Durchbohrung aufweisen, die axial vollständig durch den Nagel, nämlich von seinem ersten Nagelende zu seinem zweiten Nagelende verläuft. Insbesondere kann die Durchbohrung durch die axiale Mitte des Nagels verlaufen. Eine entsprechende Durchbohrung wird häufig als Kanülierung bezeichnet. Eine Kanülierung kann insbesondere den Zweck erfüllen, dass der Nagel zur Implantation eines erfindungsgemäßen Implantats über einen Führungsdraht implantierbar ist. Der Führungsdraht kann in der Kanülierung durch den Nagel verlaufen, so dass der Nagel zur Implantation über den Führungsdraht geschoben werden und gezielt implantiert werden kann.

Ferner wird ein Verfahren zur Implantation eines erfindungsgemäßen Implantats zur Stabilisierung von Beckenringfrakturen beschrieben, jedoch nicht beansprucht, wobei ein Durchführungskanal in oder durch ein Sacrum vorgebohrt wird, wobei ein Nagel, der vom Implantat umfasst ist, durch den Durchführungskanal durch das Sacrum durchgeführt oder durch den Durchführungskanal in das Sacrum eingeführt wird, wobei der Nagel nach dem Durchführen oder Einführen an seinem zweiten Ende in einem ersten Beckenknochen und/oder dem Sacrum fixiert wird, wobei nach dem Fixieren des Nagels an seinem zweiten Ende von außen ein Kompressionsdruck auf den Beckenring aufgebracht wird, durch welchen Druck der Beckenring im Bereich der Längserstreckung des Nagels komprimiert wird, wobei der Nagel an seinem ersten Ende in einem zweiten Beckenknochen so verankert wird, dass der Nagel relativ zu dem zweiten Beckenknochen gegen Verdrehungen und Verschiebungen gesichert ist. Vorteilhafterweise erfolgt die Verankerung des Nagels in dem zweiten Beckenknochen, nachdem der Kompressionsdruck aufgebracht worden ist. Vorteilhafterweise wird der Kompressionsdruck während der Verankerung des Nagels in dem zweiten Beckenknochen unverändert aufrechterhalten. Vorteilhafterweise wird der Kompressionsdruck zumindest in einer Komponente in Richtung der axialen Erstreckung des Nagels aufgebracht. In Bezug auf die Ausgestaltung der genannten Bauteile sei vollinhaltlich auf die übrigen Ausführungen zu der Erfindung verwiesen.

In einer Ausführungsform erfolgt die Implantation unilateral, wobei der Nagel in den in das Sacrum vorgebohrten Durchführungskanal eingeführt wird und in dem Sacrum an seinem zweiten Nagelende gegen eine Verschiebung in Richtung seiner axialen Erstreckung fixiert wird, wonach der Kompressionsdruck aufgebracht und der Nagel an seinem ersten Nagelende mittels des Verriegelungselements relativ zu dem zweiten Beckenknochen gegen Verdrehungen und Verschiebungen gesichert wird.

In einer Ausführungsform erfolgt die Implantation bilateral, wobei der Nagel durch den durch das Sacrum vorgebohrten Durchführungskanal durchgeführt wird und zumindest teilweise außerhalb des Sacrums in dem ersten Beckenknochen an seinem zweiten Nagelende gegen eine Verschiebung in Richtung seiner axialen Erstreckung fixiert wird, wonach der Kompressionsdruck aufgebracht und der Nagel an seinem ersten Nagelende mittels des Verriegelungselements relativ zu dem zweiten Beckenknochen gegen Verdrehungen und Verschiebungen gesichert wird.

Die Erfindung wird im Folgenden durch die Beschreibung von Ausführungsformen unter Bezugnahme auf die beiliegenden Figuren näher erläutert.

Es zeigen:
- Fig. 1:: eine Prinzipdarstellung eines erfindungsgemäßen Implantats;
- Fig. 2:: eine Prinzipdarstellung eines erfindungsgemäßen Verriegelungselements;
- Fig. 3:: eine Prinzipdarstellung von Front- und Seitenansicht eines menschlichen Beckenrings;
- Fig. 4:: eine Prinzipdarstellung der Frontansicht eines menschlichen Beckenrings mit einem implantierten erfindungsgemäßen Implantat;
- Fig. 5:: eine Prinzipdarstellung einer Seitenansicht eines menschlichen Beckenrings mit zwei implantierten erfindungsgemäßen Implantaten;
- Fig. 6:: eine Prinzipdarstellung einer Seitenansicht eines menschlichen Beckenrings mit zwei implantierten erfindungsgemäßen Implantat;
- Fig. 7:: eine Prinzipdarstellung der Seitenansicht auf ein menschliches Becken mit einem implantierten erfindungsgemäßen Implantat gemäß der Frontansicht nach Fig. 6;
- Fig. 8:: eine Prinzipdarstellung der Frontansicht eines menschlichen Beckens mit einem erfindungsgemäßen Implantat;
- Fi. 9:: eine Prinzipdarstellung einer Seitenansicht des menschlichen Beckens mit dem unteren Ende der Lendenwirbelsäule mit einem erfindungsgemäßen Implantat, an das eine Stabilisierungsmontage der Lendenwirbelsäule angekoppelt ist:
- Fig. 10:: eine Prinzipdarstellung der Ankoppelungsvorrichtung zwischen Implantat und Stabilisierungsmontage nach Figur 9.

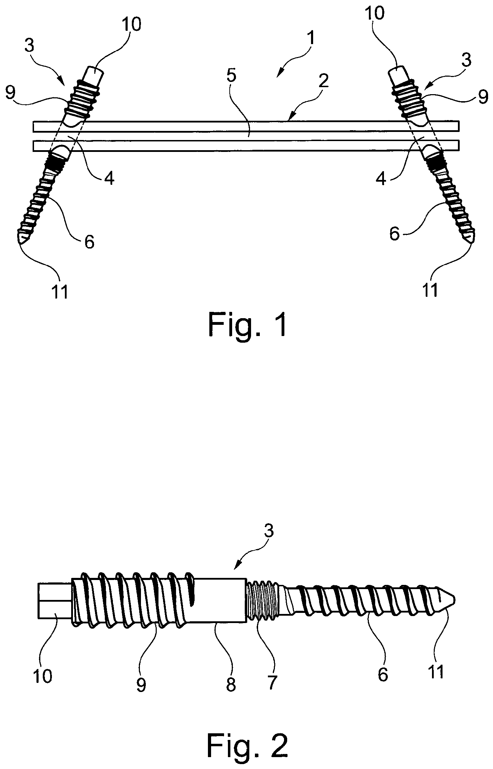

In Fig. 1 ist ein erfindungsgemäßes Implantat 1 dargestellt, das einen Nagel 2 sowie zwei Verriegelungselemente 3 umfasst. In dem Nagel 2 sind eine Kanülierung 5 und zwei transversale Durchführungen 4 angeordnet. Die Kanülierung 5 in dem Nagel 2 ist in Fig. 1 wie bei einer Schnittdarstellung dargestellt. Tatsächlich ist der Nagel 2 des in Fig. 1 dargestellten erfindungsgemäßen Implantats so ausgebildet, dass er nach Art eines langgestreckten Zylinders gestaltet ist, wobei der Zylinder als Kanülierung 5 eine durch die Längserstreckungsachse des Zylinders führende Durchbohrung aufweist. Diese Durchbohrung, und damit der Durchmesser der Kanülierung 5, beträgt in dem dargestellten Ausführungsbeispiel 3,2 mm. In anderen Ausführungsbeispielen können entsprechende Kanülierungen 5 mit anderen Durchmessern vorgesehen sein, beispielsweise mit einem Durchmesser, der zwischen 1 mm und 5 mm liegt. Bei der Wahl des Durchmessers der Kanülierung 5 kann insbesondere auf den Durchmesser des Nagels 2 und/oder den Durchmesser eines für die Implantation des erfindungsgemäßen Implantats erforderlichen Führungsdrahts abgestellt werden.

Die transversalen Durchführungen 4 in dem Nagel 2 sind in Fig. 1 durch gestrichelte Linien dargestellt. Die transversalen Durchführungen 4 durchqueren demnach den Nagel 2 über seinen gesamten Durchmesser und sind als gerader Kanal ausgebildet. In anderen Ausführungsformen des erfindungsgemäßen Implantats können die transversalen Durchführungen 4 auch entlang ihrer Erstreckungsrichtung einen gekrümmten Verlauf aufweisen, mit dem beispielsweise ein Verklemmen und damit winkelstabiles Fixieren zwischen einem Verriegelungselement 3 und dem Nagel 2 unterstützt sein kann. Die Erstreckungsrichtung einer jeden der beiden transversalen Durchführungen 4 des Nagels 2 bildet einen Winkel zur Senkrechten zur Erstreckungsachse es Nagels. Dieser Winkel ist für beide transversalen Durchführungen 4 identisch und beträgt etwa 20°. Die Erstreckungsrichtungen beider transversalen Durchführungen 4 spannen eine Ebene auf, sodass die beiden Erstreckungsrichtungen zwar in Richtung der Erstreckungsachse des Nagels 2, nicht aber in Richtung der Senkrechten zur Erstreckungsachse des Nagels 2 einen Winkel von größer 0° bilden. In anderen, in Fig. 1 nicht dargestellten Ausführungsformen, ist es selbstverständlich auch möglich, dass die Erstreckungsrichtungen der transversalen Durchführungen 4, die in einem Nagel 2 vorgesehen sind, sowohl in Richtung der Erstreckungsachse des Nagels 2 als auch in Richtung der Senkrechten zur Erstreckungsachse des Nagels 2 oder auch nur in Richtung der Senkrechten zur Erstreckungsachse des Nagels 2 einen Winkel von größer 0° bilden. Es ist somit möglich, dass die Erstreckungsrichtungen der transversalen Durchführungen 4 nicht in einer Ebene angeordnet sind.

Eine detaillierte Darstellung der Verriegelungselemente 3, die von dem erfindungsgemäßen Implantat nach Fig. 1 umfasst sind, ist in Fig. 2 gegeben. Die beiden Verriegelungselemente 3 in dem Implantat 1 nach Fig. 1 sind jeweils identisch ausgebildet. In anderen, nicht dargestellten Ausführungsformen können die in einem erfindungsgemäßen Implantat 1 vorgesehenen Verriegelungselemente 3 auch zumindest teilweise unterschiedlich ausgebildet sein. Es ist lediglich erforderlich, dass jeweils ein Verriegelungselement 3 zumindest mit jeweils einer der transversalen Durchführungen 4 korrespondiert.

Das erfindungsgemäße Verriegelungselement 3, das in dem erfindungsgemäßen Implantat 1 nach Fig. 1 zum Einsatz kommt, weist einen ersten Verankerungsabschnitt 6, einen Verriegelungsabschnitt 7, einen Zapfenabschnitt 8 und einen zweiten Verankerungsabschnitt 9 auf. An seinem ersten Ende 11 ist das Verriegelungselement 3 konisch abgeflacht, sodass die Einführung des Verriegelungselements 3 in eine transversale Durchführung 4 des Nagels 2 einfach realisierbar ist. An seinem zweiten Ende weist das Verriegelungselement 3 einen Werkzeuganschluss 10 auf. Der Werkzeuganschluss 10 ist nach Art eines Sechskants ausgebildet, sodass das Verriegelungselement 3 mittels eines Sechskantschraubwerkzeugs implantiert werden kann.

Das Verriegelungselement 3 ist nach Art einer Schraube mit verschiedenen Gewindeabschnitten ausgebildet. Der erste Verankerungsabschnitt 6 weist ein Knochengewinde auf, mit dem das Verriegelungselement 3 nach der Durchführung durch die zugeordnete transversale Durchführung 4 in einen Knochen schraubbar ist. Auf den ersten Verankerungsabschnitt 6 folgt der Verriegelungsabschnitt 7, der in dem dargestellten Ausführungsbeispiel von dem ersten Verankerungsabschnitt 6 abgesetzt ist und ein metrisches Gewinde aufweist. Der Durchmesser des Verriegelungselements 3 in dem Verriegelungsabschnitt 7 ist größer als der Durchmesser in dem ersten Verankerungsabschnitt 6. Dabei ist der Durchmesser des Verriegelungsabschnitts 7 an den Innendurchmesser der dem Verriegelungselement 3 zugeordneten transversalen Durchführung 4 eines Nagels 2 angepasst. Die zugeordnete transversale Durchführung 4 weist ein metrisches Gewinde auf, das mit dem an dem Verriegelungsabschnitt 7 vorgesehenen metrischen Gewinde korrespondiert. Somit erfolgt die Verriegelung zwischen Nagel 2 und Verriegelungselement 3 über eine Verschraubung des Verriegelungselements 3 über das metrische Gewinde im Verriegelungsabschnitt 7 mit dem metrischen Gewinde in der transversalen Durchführung 4 des Nagels 2.

Da der Durchmesser des Verriegelungselements 3 in dem ersten Verankerungsabschnitt 6 kleiner ist als in dem Verriegelungsabschnitt 7, kann das Verriegelungselement 3 mit seinem ersten Ende 11 zuerst durch die transversale Durchführung 4 durchgeführt werden, ohne dass das Knochengewinde im ersten Verankerungsabschnitt 6 und das metrische Gewinde in der transversalen Durchführung 4 sich gegenseitig Schaden zufügen. Sodann erfolgt die Fixierung zwischen Nagel 2 und Verriegelungselement 3 durch Verschraubung des Verriegelungsabschnitts 7 mit der transversalen Durchführung 4. Vorteilhafterweise wird das Verriegelungselement 3 während des gesamten Durchführungsvorgangs durch die transversale Durchführung 4 durchgehend gedreht, damit das Knochengewinde am ersten Verankerungsabschnitt 6 sich in einen Knochen schrauben kann, sobald das erste Ende 11 des Verriegelungselements 3 auf einen Knochen stößt. Bei einem solchen Drehvorgang kann die Verankerung des Verriegelungselements 3 gleichzeitig mit der Verriegelung erfolgen.

Die winkelstabile Verriegelung zwischen Verriegelungselement 3 und Nagel 2 wird in dem in Fig. 1 dargestellten Ausführungsbeispiel insbesondere dadurch unterstützt, dass vom ersten Ende 11 des Verriegelungselements 3 aus gesehen hinter dem Verriegelungsabschnitt 7 ein Zapfenabschnitt 8 vorgesehen ist, wobei der Durchmesser des Zapfenabschnitts 8 größer ist als der Durchmesser des Verriegelungsabschnitts 7. Bei dem Verriegelungsvorgang zwischen Verriegelungselement 3 und Nagel 2 wird der Zapfenabschnitt 8 gegen das metrische Gewinde, das in der transversalen Durchführung 4 angeordneten ist, gepresst. Da der Durchmesser des Zapfenabschnitts 8 größer ist als der Innendurchmesser des Gewindes in der transversalen Durchführung 4, kann der Zapfenabschnitt 8 nicht durch die transversale Durchführung 4 gleiten, sondern es entsteht eine sehr starre, winkelstabile Verbindung zwischen Nagel 2 und Verriegelungselement 3. In der beschriebenen vorteilhaften Ausführungsform ist die transversale Durchführung 4 so ausgebildet, dass sie an dem Ende, das dem Zapfenabschnitt 8 zugeordnet ist, einen Abschnitt ohne Gewinde aufweist, der den Zapfenabschnitt 8 umfänglich formschlüssig aufnehmen kann, so dass eine besonders stabile Fixierung zwischen Nagel 2 und Verriegelungselement 3 erzielbar ist.

Vom ersten Ende 11 des Verriegelungselements 3 aus gesehen hinter dem Zapfenabschnitt 8 ist ein zweiter Verankerungsabschnitt 9 angeordnet. Der zweite Verankerungsabschnitt 9 weist ein Knochengewinde auf. Bei dem Drehen des Verriegelungselements 3 zur Verankerung des Verriegelungselements 3 mittels des ersten Verankerungsabschnitts 6 und zur Verriegelung mittels des Verriegelungsabschnitts 7 wird das Verriegelungselement 3 gedreht. Dabei wird auch der zweite Verankerungsabschnitt 9 in einem Knochen verankert, indem das Knochengewinde an dem zweiten Verankerungsabschnitt 9 sich in einen Knochen einschneidet. Somit ist nach der abgeschlossenen Durchführung des Verriegelungselements 3 durch die transversale Durchführung 4 des Nagels 2 das Verriegelungselement 3 in seinem ersten Verankerungsabschnitt 6 und seinem zweiten Verankerungsabschnitt 9 jeweils in einem Knochen verankert. Zudem ist das Verriegelungselement 3 dann mit dem Nagel 2 verriegelt. Die Verankerungen sind, wie aus Fig. 1 ersichtlich, oberhalb und unterhalb der Verriegelung zwischen Verriegelungselement 3 und Nagel 2 angeordnet. Dadurch kann eine besonders gute Fixierung des mit dem Verriegelungselement 3 winkelstabil fixierten Nagels 2 in einem Beckenknochen gewährleistet werden.

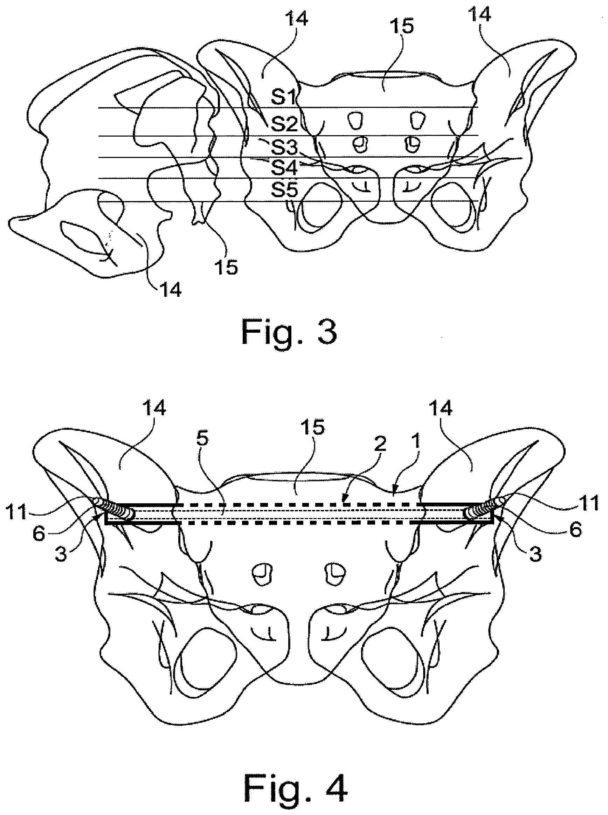

In Fig. 3 ist das menschliche Becken umfassend das Sacrum 15 und jeweils an einer Seite des Sacrums 15 angeordnet ein Ilium 14 dargestellt. Das Sacrum 15 umfasst fünf miteinander verschmolzene Wirbel. Entsprechend weist das Sacrum 15 fünf Bereiche auf, in denen das Sacrum 15 über seine gesamte Breite hinweg als durchgehender Knochen ausgebildet ist. Diese Bereiche werden mit S1-, S2-, S3- S4- und S5-Korridor bezeichnet. Diese Bereichsbezeichnung ist in Fig. 3 dargestellt. Ein erfindungsgemäßes Implantat wird vorzugsweise in einem der genannten fünf Bereiche bzw. Korridore implantiert. Bei einem Bruch des Sacrums oder des Iliosakralgelenks kann ein erfindungsgemäßes Implantat so in dem Beckenring implantiert werden, dass der Nagel 2 intraossär durch das Sacrum 15 in einem der genannten Korridore implantiert ist, wobei sich der Nagel 2 an beiden Seiten über das Sacrum hinaus erstreckt, sodass die beiden Enden des Nagels 2 jeweils in einem Ilium verankerbar sind.

In Fig. 4 ist ein menschlicher Beckenring nach Fig. 3 mit einem erfindungsgemäßen Implantat 1 gemäß Fig. 1 dargestellt. Das erfindungsgemäße Implantat 1 wurde durch den S1-Korridor bereichsweise innerhalb des Sacrums 15 implantiert. Der Nagel 2 des Implantats 1 erstreckt sich zu beiden Seiten des Sacrums 15 über das Sacrum hinaus, sodass eine Verankerung des Nagels 2 an seinen beiden Enden in einem Ilium 14 über die Kombination zwischen einer transversalen Durchführung 4 und einem Verriegelungselement 3 realisierbar ist. In dem in Fig. 4 gezeigten Beispiel sind die Verriegelungselemente 3 jeweils von hinten nach vorne durch das Ilium geschraubt. Aus Fig. 4 ist offensichtlich, dass die Verriegelungselemente 3 selbst während der Implantation keinen oder zumindest nur einen sehr geringen Druck auf Knochen in dem Beckenring in einer Richtung entlang der Erstreckungsachse des Nagels 2 ausüben. Vielmehr dienen die Verriegelungselemente 3 im Wesentlichen ausschließlich der Verankerung des Nagels 2 in einem Ilium zur winkelstabilen Fixierung zwischen Nagel 2 und Ilium 14. Die Implantationen des Implantats 1 zur Realisierung einer wie in Fig. 4 dargestellten Implantation kann beispielsweise dadurch erfolgen, dass zuerst durch den S1-Korridor des Sacrum 15 ein Durchführungskanal gebohrt wird, sodann der Nagel 2 durch den Durchführungskanal durchgeführt wird, der Nagel 2 dann an seinem zweiten Ende mit einem ersten Ilium 14 mittels des Verriegelungselements 3 in Kombination mit der transversalen Durchführung 4 fixiert wird, dann eine Kraft von außen auf das Becken aufgebracht wird, um Knochenfragmente zusammenzupressen und in ihre richtige Position zu bringen, wonach dann der Nagel 2 an seinem ersten Ende mit einem weiteren Verriegelungselement 3 in Kombination mit einer weiteren transversalen Durchführung 4 in dem zweiten Ilium 14 verankert wird. Für den Fachmann ist ersichtlich, dass das erfindungsgemäße Implantat insbesondere bei Brüchen des Sacrums 15 und des Iliosakralgelenks vorteilhaft ist, da die Fragmente des Sacrums 15 bzw. des Iliosakralgelenks durch die feste, winkelstabile Fixierung der beiden Ilia 14 zueinander sehr gut stabilisierbar sind, sodass eine sehr gute Heilung entsprechender Brüche ermöglicht ist.

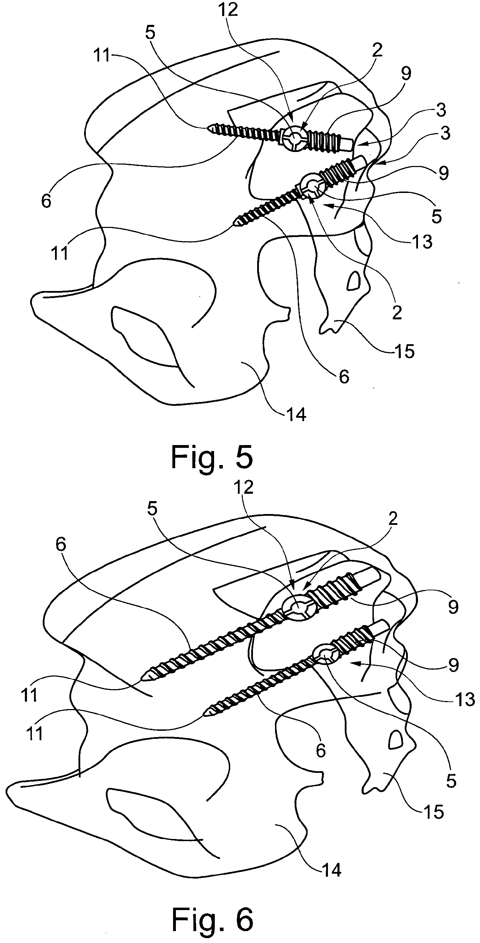

In Fig. 5 ist eine Seitenansicht eines menschlichen Beckens mit zwei implantierten erfindungsgemäßen Implantaten 12, 13 dargestellt. Das in Fig. 5 oben gelegene Implantat 12 ist durch den S1-Korridor des Sacrums 15 implantiert, das in Fig. 5 unten gelegene Implantat 13 ist durch den S2-Korridor des Sacrums 15 implantiert. Die Erstreckungsrichtung der Verriegelungselemente 3 des oben gelegenen Implantats 12 ist zur Erstreckungsrichtung der Verriegelungselemente 3 des unten gelegenen Implantats 13 verkippt. Dabei ist zu berücksichtigen, dass die Erstreckungsrichtung der Verriegelungselemente 3 bzw. die Einschraubrichtung der Verriegelungselemente 3 bei der Implantation jeweils nach medizinischen Gesichtspunkten frei wählbar ist. Eine medizinische Indikation für die Einschraubrichtung der Verriegelungselemente 3 kann beispielsweise sein, dass die Verriegelungselemente 3 mit ihren Verankerungsabschnitten 6, 9 in möglichst stabile Knochensubstanz geschraubt werden. Darüber hinaus können auch Nervenverläufe oder andere medizinische Indikationen bei dem Ausrichten der Verriegelungselemente 3 eine Rolle spielen. In Fig. 5 ist der Querschnitt des Nagels erkennbar. Der Nagel weist eine Kanülierung 5 auf und ist um die Kanülierung 5 als Hohlzylinder ausgebildet. In Fig. 5 ist darüber hinaus die Koppelvorrichtung des Nagels dargestellt, die dem Ankoppeln eines Zielgeräts dient.

In Figur 6 ist ein weiteres Beispiel einer in einem Beckenring erfolgten Implantation von erfindungsgemäßen Implantaten 12, 13 dargestellt.

Das Implantat 12 ist in der in Figur 6 dargestellten Implantationssituation so implantiert, dass der Nagel 2 des Implantats 12 durch den S1-Korridor des Sacrums 15 verläuft. Im Vergleich zu der in den Figuren 4 und 5 dargestellten Implantationssituation eines Implantats 1, 12 in dem S1-Korridor des Sacrums 15 sind die Verriegelungselemente 3 mit einer anderen Erstreckungsrichtung orientiert. Das Implantat 13 ist im Vergleich zu der in Figur 5 dargestellten Implantationssituation nur leicht verkippt implantiert. Im Vergleich zu den in Figur 5 dargestellten Implantaten 12, 13 weisen die Verriegelungselemente 3 der in Figur 6 dargestellten Implantate 12, 13 unterschiedliche Längen auf. Es ist ersichtlich, dass der Fachmann die jeweiligen geometrischen Merkmale von Verriegelungselementen 3 und Implantaten 1, 12, 13 im Hinblick auf die jeweils für das erfindungsgemäße Implantat 1, 12, 13 vorgesehene Implantationssituation anpassen kann.

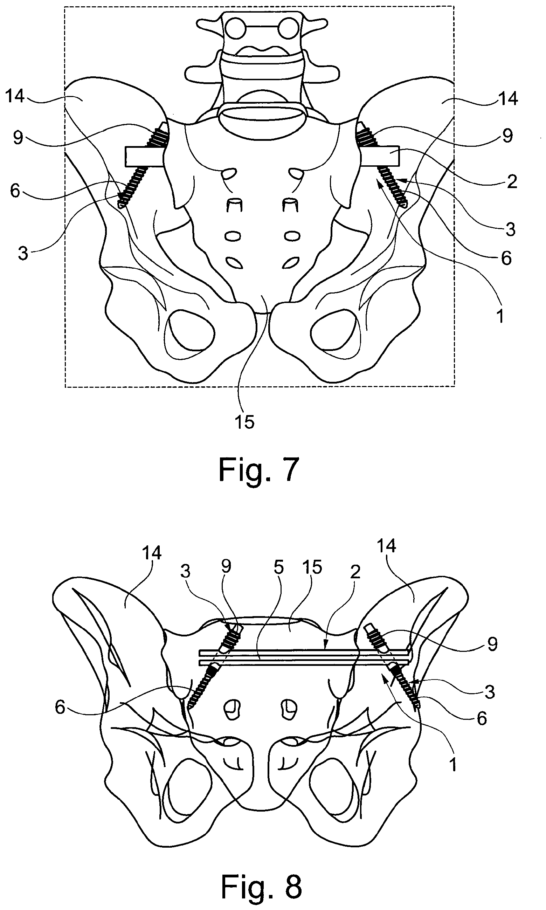

In Figur 6 sind die Verriegelungselemente 3 bei beiden Implantaten 12, 13 vom menschlichen Körper aus gesehen von hinten oben nach vorne unten verschraubt. Der zweite Verankerungsabschnitt der Verriegelungselemente 3 kann dabei nicht nur in dem Ilium 14, sondern auch in dem Sacrum 15 verankert sein. Dadurch kann eine besonders gute Stabilisierung des Sacrums 15 gegenüber dem Ilium 14 und eine besonders gute Verankerung des Nagels 2 in dem Beckenring insgesamt erreichbar sein. Eine entsprechende Verankerung der Verriegelungselemente 3 in dem Sacrum 15 und in dem Ilium 14 ist in Figur 7 erkennbar. In Figur 7 ist eine Frontansicht eines menschlichen Beckenrings dargestellt, in dem ein erfindungsgemäßes Implantat 1 bereichsweise in dem S1-Korridor des Sacrums 15 implantiert ist. Die in Figur 7 dargestellte Implantationssituation des Implantats 1 entspricht der in Figur 6 dargestellten Implantationssituation des Implantats 12.

In Figur 8 ist die Frontansicht eines menschlichen Beckens mit einer Ausführungsform des erfindungsgemäßen Implantats 1 dargestellt. Im Vergleich zu den Ausführungsbeispielen, die in den Figuren 4 bis 7 dargestellt sind, ist bei dem erfindungsgemäßen Implantat 1 nach Figur 8 ein kürzerer Nagel 2 vorgesehen. Das erfindungsgemäße Implantat umfasst zwei Verriegelungselemente 3 und der Nagel 2 des Implantats 1 weist zwei transversale Durchführungen 4 auf. Das Fixierungsmittel des erfindungsgemäßen Implantats 1 ist durch das Zusammenwirken eines der beiden Verriegelungselemente 3 mit einer der beiden transversalen Durchführungen 4 realisiert. In Figur 8 ist das erfindungsgemäße Implantat 1 unilateral eingesetzt. Der Nagel 2 ist durch einen in dem Sacrum 15 vorgebohrten Durchführungskanal intraossär in das Sacrum 15 eingeführt. Der Nagel 2 ist mittels eines der beiden Verriegelungselemente 3 in dem Sacrum 15 fixiert. Weiterhin ist der Nagel 2 mittels des anderen der beiden Verriegelungselemente 3 in einem Illium 14 verankert. Bei der Implantation des Implantats 1 nach Figur 8 wird zuerst der Nagel 2 in den Durchführungskanal in dem Sacrum 15 eingeführt und dann mit einem Verriegelungselement 3 in dem Sacrum 15 an seinem zweiten Nagelende fixiert. Daraufhin wird auf den Beckenring von außen ein Kompressionsdruck aufgebracht, beispielsweise über eine Kompressionszwinge oder ein Zielgerät. Die Kompressionszwinge oder das Zielgerät können an dem ersten Nagelende, das nach dem Einführen des Nagels 2 durch den Durchführungskanal in dem Sacrum 15 und nach dem Fixieren des Nagels 2 in dem Sacrum 15 in einem Illiumbereich liegt, angekoppelt sein. Dadurch kann gewährleistet sein, dass nach dem Aufbringen des Kompressionsdrucks von außen auf den Beckenring das zweite der beiden Verriegelungselemente 3 durch die zweite der beiden transversalen Durchführungen 4 zielsicher durchgeführt werden kann und in dem Illium 14 verankert werden kann. Durch das in Figur 8 dargestellte erfindungsgemäße Implantat 1 lässt sich eine winkelstabile Fixierung des Sacrums 15 zu einem Illiumknochen 14 realisieren. In Figur 8 ist der Nagel 2 durch einen Durchführungskanal in dem S1-Korridor des Sacrums 15 in das Sacrum 15 eingeführt. Je nach den spezifischen Gegebenheiten des Einzelfalls, insbesondere im Hinblick auf die Art und die Stelle des Bruchs sowie die Ausgestaltung des menschlichen Beckenrings, in dem das erfindungsgemäße Implantat 1 nach Figur 8 zu implantieren ist, kann das Implantat 1 in verschiedenen Positionen in dem Beckenring implantiert sein und die Erstreckungsrichtung der transversalen Durchführungen 4 in dem Nagel 2 können nach Bedarf vorgesehen sein. Auf das Implantat 1 nach Figur 8, das für den unilateralen Einsatz geeignet ist, sind die Ausgestaltungs- und Implantationsmöglichkeiten, die in der Beschreibung zu den Figuren 1 bis 7 geschildert sind, entsprechend anwendbar.

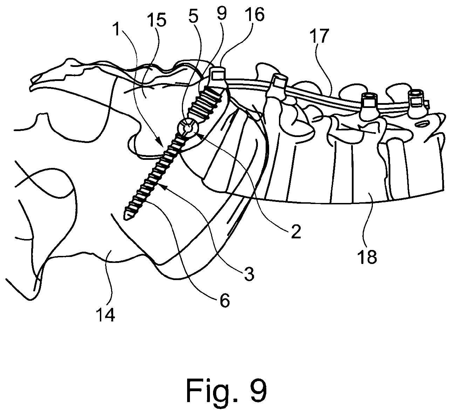

In Figur 9 ist eine Seitenansicht des menschlichen Beckens mit dem unteren Ende der Lendenwirbelsäule dargestellt, wobei in dem Becken ein erfindungsgemäßes Implantat 1 implantiert ist, an das eine Stabilisierungsmontage der unteren Lendenwirbelsäule 18 angekoppelt ist. Das in Figur 9 dargestellte Implantat 1 weist im Wesentlichen dieselben Merkmale auf wie das in Figur 6 dargestellte Implantat 12 im S1-Korridor. Darüber hinaus weist das Implantat 1 nach Figur 9 ein Verbindungselement 16 auf, über das ein Stabilisierungselement 17 einer Stabilisierungsmontage der unteren Lendenwirbelsäule 18 an das Implantat 1 angekoppelt ist. Das Verbindungselement 16 ist an dem zweiten Ende des Verriegelungselements 3 angeordnet und damit an dem Ende des Verriegelungselements 3, an dem sich der zweite Verankerungsabschnitt 9 befindet. Durch das Vorsehen der Stabilisierungsmontage für die unteren Lendenwirbelkörper und das Ankoppeln dieser Stabilisierungsmontage an das erfindungsgemäße Implantat 1 ist eine stabile Anordnung von dem Beckenring zu der unteren Lendenwirbelsäule 18 gewährleistet. Dadurch kann eine sehr gute Ruhigstellung von geschädigten Stellen in dem Beckenring und/oder der unteren Lendenwirbelsäule 18 gewährleistet sein.

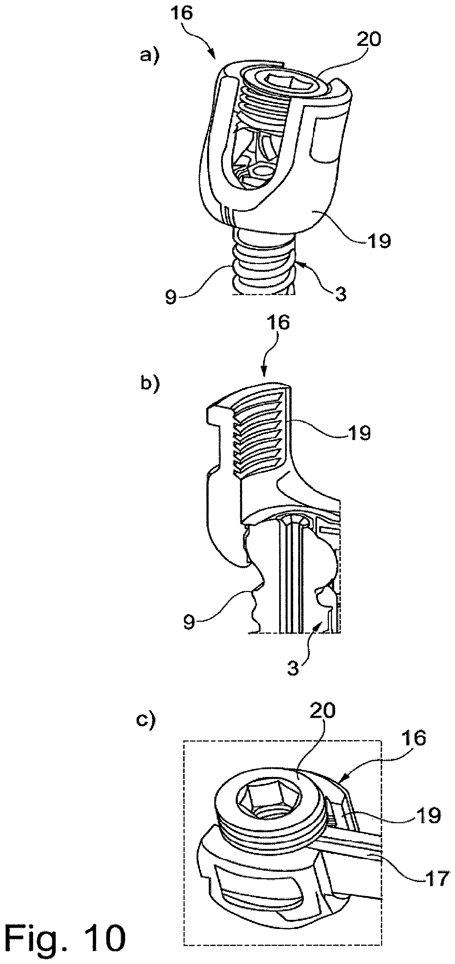

Das Verbindungselement 16, das von dem in Figur 9 dargestellten erfindungsgemäßen Implantat 1 umfasst ist, ist in den Figuren 10a), 10b) und 10c) detailliert dargestellt. Das Verbindungselement 16 umfasst einen Haltekörper 19 sowie eine Schraube 20, die in den Haltekörper 19 schraubbar ist. Entsprechend weist der Haltekörper 19 an seinem dem Verriegelungselement 3 abgewandten Ende ein Innengewinde auf. Das Verbindungselement 16 weist eine Aufnahme auf, die mit dem zweiten Ende des Verriegelungselements 3 korrespondiert. Das zweite Ende des Verriegelungselements 3 kann passgenau von der Aufnahme des Verbindungselements 16 aufgenommen werden. In dem dargestellten Ausführungsbeispiel sind die Aufnahme des Haltekörpers 19 und das zweite Ende des Verriegelungselements 3 jeweils abschnittsweise abgerundet, so dass die Erstreckungsrichtung des Verbindungselements 16 nicht parallel zu der Erstreckungsachse des Verriegelungselements 3 verlaufen muss, sondern das Verbindungselement 16 kann zum Verriegelungselement 3 verkippt angeordnet sein, siehe Figur 10a). Dadurch ist eine gewisse Flexibilität zwischen der Stabilisierungsmontage der unteren Lendenwirbelsäule und dem erfindungsgemäßen Implantat 1 gewährleistet. In dem gezeigten Ausführungsbeispiel wird ein Verkippen zwischen Verbindungselement 16 und Verriegelungselement 3 insbesondere dadurch gewährleistet, dass das Verriegelungselement 3 in der Nähe seines zweiten Endes eine umfänglich verlaufende Einkerbung aufweist, in die sich der Haltekörper 19 des Verbindungselements 16 im zum Verriegelungselement 3 verkippten Zustand hineinerstrecken kann. Andere, nicht dargestellte Ausführungsbeispiele können sich von dem in Fig. 10 dargestellten Ausführungsbeispiel beispielsweise dadurch unterscheiden, dass keine Abrundung am Verriegelungselement 3 und/oder an der Aufnahme des Haltekörpers 19 vorgesehen sind, so dass eine entsprechende Flexibilität nicht gewährleistet ist.

Der Haltekörper 19 weist eine Aussparung auf, durch die ein Stabilisierungselement 17 einer Stabilisierungsmontage durchgeführt werden kann. In Figur 10 c)ist ein Verbindungselement 16 dargestellt, durch dessen Aussparung ein Stabilisierungselement 17 durchgeführt wurde. Die Fixierung des Stabilisierungselements 17 gegenüber dem Verbindungselement 16 erfolgt dadurch, dass die Schraube 20 über das Innengewinde an dem Haltekörper 19 in den Haltekörper 19 geschraubt wird und dabei ein Anpressdruck auf das Stabilisierungselement 17 erzeugt. An dem Haltekörper 19 ist an der Außenseite ein Werkzeugansatz vorgesehen, auf den ein Werkzeug aufgesetzt werden kann, damit bei einem Einschrauben der Schraube 20 in den Haltekörper 19 ein Verdrehen des Haltekörpers 19 ausgeschlossen werden kann und somit auf die Schraube 20 ein ausreichendes Anzugsdrehmoment ausgeübt werden kann, ohne dass es zu einem Verdrehen des Haltekörpers 19 kommt. Andere Ausführungen können sich von der in Fig. 10 dargestellten Ausführungsform beispielsweise dadurch unterscheiden, dass Werkzeugansatz und/oder die Aussparung anders ausgestaltet sind.

Den beschriebenen Ausführungsbeispielen ist zu entnehmen, dass das erfindungsgemäße Implantat 1 sehr einfach und vielseitig bei einer Implantation zur Stabilisierung von Beckenringfrakturen eingesetzt werden kann. Die Ausführungsbeispiele zeigen verschiedene Beispiele dafür, wie ein erfindungsgemäßes Implantat 1, 12, 13 in einem menschlichen Beckenring implantiert werden kann. Selbstverständlich können zur Stabilisierung von Beckenringfrakturen mehrere erfindungsgemäße Implantate 1, 12, 13 eingesetzt werden. Zudem kann jede der beschriebenen Implantationssituationen, insbesondere betreffend die Lage des Implantats 1, 12, 13, bei Verwendung nur eines Implantats auf dieses Implantat bezogen realisiert werden. Auch kann ein erfindungsgemäßes Implantat nicht nur im menschlichen Beckenring, sondern beispielsweise auch bei anderen Säugetieren eingesetzt werden.

Das erfindungsgemäße Implantat 1 ermöglicht insbesondere eine Implantation unter Berücksichtigung des konkreten medizinischen Einzelfalls und eine Schonung der Kontakte von Knochenfragmenten entlang der Bruchstelle. Zudem ist durch die winkelstabile Verriegelung zwischen Verriegelungselementen 3 und Nagel 2 das Implantat 1 insgesamt in sich winkelstabil und verdrehfest, sodass durch das Implantat 1 im Besonderen eine winkelstabile Fixierung von Beckenringfrakturen ermöglicht wird. Zudem kann die Erfindung in gleicher Weise möglicherweise auch zur Durchführung einer Arthrodese bei degenerativen Instabilitäten und/oder Arthrosen des Sakroilialgelenks verwendet werden.

### Bezugszeichenliste

1 Implantat
2 Nagel
3 Verriegelungselement
4 transversale Durchführung
5 Kanülierung
6 erster Verankerungsabschnitt
7 Verriegelungsabschnitt
8 Zapfenabschnitt
9 zweiter Verankerungsabschnitt
10 Werkzeuganschluss
11 erstes Ende des Verriegelungselements
12 Implantat im S1-Korridor
13 Implantat im S2-Korridor
14 Ilium
15 Sacrum
16 Verbindungselement
17 Stabilisierungselement
18 untere Lendenwirbelsäule
19 Haltekörper
20 Schraube

## Patentansprüche

1. Implantat (1) zur Stabilisierung von Beckenringfrakturen in einem Beckenring, umfassend einen Nagel (2), ein Fixierungsmittel sowie zumindest ein Verriegelungselement (3) zur relativen Fixierung von Beckenknochen zum Nagel (2), **dadurch gekennzeichnet, dass** der Nagel (2) zur intraossären Implantation ausgestaltet ist und durch einen durch den S1- oder den S2-Korridor (12, 13) des Sacrums (15) vorgebohrten Durchführungskanal intraossär durchführbar oder in einen in den S1- oder S2-Korridor (12, 13) des Sacrums (15) vorgebohrten Durchführungskanal intraossär einführbar ist, dass der Nagel (2) zumindest an seinem ersten Nagelende eine transversale Durchführung (4) zur Aufnahme des Verriegelungselements (3) aufweist, dass das Verriegelungselement (3) einen Verankerungsabschnitt (6, 9) aufweist, mittels dessen es in einem Beckenknochen (14) verankerbar ist, sowie einen Verriegelungsabschnitt (7), der mit der transversalen Durchführung (4) des Nagels (2) so korrespondiert, dass das Verrieglungselement (3) winkelstabil mit dem Nagel (2) verriegelbar ist, dass der Nagel (2) mittels des Fixierungsmittels an seinem zweiten Nagelende in einem ersten Beckenknochen (14) und/oder dem Sacrum (15) fixierbar ist, dass der Nagel derart ausgebildet ist, dass nach dessen Fixierung an seinem zweiten Ende unter Aufbringung eines Kompressiondruckes von außen auf den Beckenring der Beckenring im Bereich der Längserstreckung des Nagels komprimierbar ist, dass der Nagel (2) mittels des Verriegelungselements (3) relativ zu einem zweiten Beckenknochen gegen Verdrehungen und Verschiebungen gesichert fixierbar ist, und dass der Nagel eine Länge von 80 mm bis 220 mm aufweist.

2. Implantat (1) nach Anspruch 1, **dadurch gekennzeichnet, dass** der Nagel (2) so ausgestaltet ist, dass der durch einen durch den S1- oder den S2-Korridor (12, 13) des Sacrums (15) vorgebohrten Durchführungskanal intraossär durchführbare Nagel (2)eine Länge von 140 mm bis 180 mm aufweist und/oder dass der in einen in den S1- oder S2-Korridor (12, 13) des Sacrums (15) vorgebohrten Durchführungskanal intraossär einführbare Nagel (2) eine Länge von 80 bis 140 mm aufweist.

3. Implantat (1) nach einem der vorangehenden Ansprüche, **dadurch gekennzeichnet, dass** die transversale Durchführung (4) ein Innengewinde aufweist, das mit einem an dem Verriegelungsabschnitt (7) des Verriegelungselements (3) vorgesehenen Gewinde korrespondiert, und/oder dass zumindest an dem Verankerungsabschnitt (6, 9) des Verriegelungselements (3) ein Knochenschraubengewinde vorgesehen ist und/oder dass der Durchmesser des Verriegelungselements (3) in dem Verriegelungsabschnitt (7) größer ist als in dem Verankerungsabschnitt (6).

4. Implantat (1) nach einem der vorangehenden Ansprüche, **dadurch gekennzeichnet, dass** der Verankerungsabschnitt (6) an einem ersten Ende des Verriegelungselements (11) angeordnet ist, wobei der Verriegelungsabschnitt (7) von dem ersten Ende des Verriegelungselements (11) aus gesehen hinter dem Verankerungsabschnitt (6) angeordnet ist, wobei entsprechend gesehen hinter dem Verriegelungsabschnitt (7) ein Abschnitt des Verriegelungselements (3) angeordnet ist, der zapfenartig ausgebildet ist und dessen Durchmesser zumindest genauso groß ist wie der Außendurchmesser des Verriegelungselements (3) im Verriegelungsabschnitt (7).

5. Implantat (1) nach einem der vorangehenden Ansprüche, **dadurch gekennzeichnet, dass** zumindest eines der Verriegelungselemente (3) einen zweiten Verankerungsabschnitt (9) aufweist, wobei der Verriegelungsabschnitt (7) zwischen dem ersten und dem zweiten Verankerungsabschnitt (6, 9) angeordnet ist, und wobei der zweite Verankerungsabschnitt (9) nach Art eines Knochengewindes ausgestaltet ist.

6. Implantat (1) nach Anspruch 8, **dadurch gekennzeichnet, dass** der Durchmesser des Verriegelungselements (3) in dem zweiten Verankerungsabschnitt (9) größer ist als der Durchmesser des Verriegelungselements (3) in dem ersten Verankerungsabschnitt (6).

7. Implantat (1) nach einem der vorangehenden Ansprüche, **gekennzeichnet durch** zumindest zwei Verriegelungselemente (3), wobei der Nagel (2) zumindest zwei transversale Durchführungen (4) zur Aufnahme jeweils eines der Verriegelungselemente (3) aufweist, wobei das Fixierungsmittel zur Fixierung des Nagels (2) an seinem zweiten Nagelende eines der zumindest zwei Verriegelungselemente (3) umfasst, wobei zwischen zumindest zwei der transversalen Durchführungen (4) ein Abstand vorgesehen ist, der mindestens 70% der Länge des Nagels (2) beträgt.

8. Implantat (1) nach einem der vorangehenden Ansprüche, **dadurch gekennzeichnet, dass** zumindest eine oder sämtliche der transversalen Durchführungen (4) eine Erstreckungsrichtung durch den Nagel (2) aufweist, die einen Winkel von größer 0° zur Senkrechten zur Erstreckungsachse des Nagels (2) bildet.

9. Implantat (1) nach einem der vorangehenden Ansprüche, **dadurch gekennzeichnet, dass** der Nagel (2) zumindest zwei transversale Durchführungen (4) aufweist, wobei die Erstreckungsrichtung der ersten transversalen Durchführung (4) mit der Erstreckungsrichtung der zweiten transversalen Durchführung (4) in Richtung der Erstreckungsachse des Nagels (2) und/oder in Richtung der Senkrechten zur Erstreckungsachse des Nagels (2) einen Winkel von größer 0° bildet.

10. Implantat (1) nach Anspruch 9, **dadurch gekennzeichnet, dass** die Erstreckungsrichtung der ersten transversalen Durchführung (2) mit der Erstreckungsrichtung der zweiten transversalen Durchführung (2) in Richtung der Erstreckungsachse des Nagels (2) einen Winkel zwischen 5° und 175° bildet, wobei die Durchführungen (2) an der Seite des Nagels (2), von der aus die Verriegelungselemente (3) in die Durchführungen (2) einführbar sind, weniger weit voneinander entfernt sind als auf der gegenüberliegenden Seite des Nagels (2).

11. Verriegelungselement (3) zur Verwendung in einem Implantat (1) nach einem der Ansprüche 1 bis 10, **gekennzeichnet durch** einen Verankerungsabschnitt (6) und einen Verriegelungsabschnitt (7), wobei der Verankerungsabschnitt (6) an einem ersten Ende des Verriegelungselements (11) angeordnet ist und das Verriegelungselement (3) mittels des Verankerungsabschnitts (6) mit einem Beckenknochen (14) und/oder Sacrum (15) verankerbar ist, wobei der Verriegelungsabschnitt (7) von dem ersten Ende des Verriegelungselements (11) aus gesehen hinter dem Verankerungsabschnitt (6) angeordnet ist, wobei der Verriegelungsabschnitt (7) ein Außengewinde aufweist, wobei von dem ersten Ende aus gesehen hinter dem Verriegelungsabschnitt (7) ein Abschnitt angeordnet ist, der zapfenartig ausgebildet ist und dessen Durchmesser zumindest genauso groß ist wie der Außendurchmesser des Verriegelungselements (3) im Verriegelungsabschnitt (7) ist.

12. Verriegelungselement (3) nach Anspruch 11, **dadurch gekennzeichnet, dass** der Durchmesser des Verriegelungselements (3) in dem zweiten Verankerungsabschnitt (9) größer ist als der Durchmesser des Verriegelungselements (3) in dem ersten Verankerungsabschnitt (6) und/oder in dem Verriegelungsabschnitt (7).

13. Verriegelungselement (3) nach einem der Ansprüche 11 oder 12, **gekennzeichnet durch** ein Verbindungselement (16) an seinem zweiten Ende zur Ankopplung des Verriegelungselements (3) an eine Stabilisierungsmontage (17) der unteren Lendenwirbelkörper.

14. Verriegelungselement (3) nach einem der Ansprüche 11 bis 13, **dadurch gekennzeichnet, dass** das Verriegelungselement (3) axial durchbohrt ist und/oder dass das Verriegelungselement (3) zumindest eine Querbohrung aufweist.

15. Nagel (2) zur Verwendung in einem Implantat (1) zur Stabilisierung von Beckenringfrakturen in einem Beckenring nach einem der Ansprüche 1 bis 10, **dadurch gekennzeichnet, dass** der Nagel (2) einen Durchmesser von 7 mm bis 10 mm und eine Länge von 80 mm bis 220 mm aufweist und der Nagel (2) zumindest eine transversale Durchführung (4) an seinem ersten Nagelende aufweist zur Aufnahme eines korrespondierenden Verriegelungselements, wobei der Nagel (2) so ausgebildet ist, dass er mit seinem zweiten Nagelende durch einen in einen in den S1- oder S2-Korridor (12, 13) des Sacrums (15) vorgebohrten Durchführungskanal intraossär in das Sacrum (15) einführbar und in dem Sacrum (15) fixierbar oder durch einen durch den S1- oder den S2-Korridor (12, 13) des Sacrums (15) vorgebohrten Durchführungskanal durchführbar und in einem ersten Beckenknochen (14) fixierbar ist, ohne eine Fixierung der Beckenringfraktur in Nagellängsrichtung zu gewährleisten, und dann eine Kraft in Nagellängsrichtung zur lateralen Druckbeaufschlagung von außen auf den Beckenring unter Kompression des Beckenrings im Bereich der Längserstreckung des Nagels ausübbar ist, wobei der Nagel (2) mittels des Verriegelungselements (3) relativ zu einem zweiten Beckenknochen gegen Verdrehungen und Verschiebungen gesichert fixierbar ist unter winkelstabiler Aufrechterhaltung der Kraft auf den Beckenring im Bereich der Längserstreckung des Nagels.

16. Nagel (2) nach Anspruch 15, **dadurch gekennzeichnet, dass** der Nagel (2) zumindest zwei transversale Durchführungen (4) aufweist, wobei zwischen den transversalen Durchführungen (4) ein Abstand vorgesehen ist, der mindestens 70% der Länge des Nagels (2) beträgt und/oder dass der Nagel (2) axial durchbohrt ist.

17. Nagel (2) nach einem der Ansprüche 15 oder 16, **dadurch gekennzeichnet, dass** der Nagel (2) zumindest an einem seiner beiden Enden eine Koppelvorrichtung zum Ankoppeln einer Kompressionszwinge und/oder eines Zielgeräts aufweist.

## Claims

1. Implant (1) for stabilizing pelvic ring fractures in a pelvic ring, comprising a nail (2), a fixation means and at least one locking element (3) for relative fixation of pelvic bones to the nail (2), **characterized in that** the nail (2) is designed for intraosseous implantation and can be passed intraosseously through a through-channel pre-drilled through an S1 or S2 corridor (12, 13) of the sacrum (15) or can be inserted intraosseously in a through-channel pre-drilled in the S1 or S2 corridor (12, 13) of the sacrum (15), that the nail (2) has, at least at its first nail end, a transverse passage (4) for receiving the locking element (3), that the locking element (3) has an anchoring portion (6, 9) by means of which it can be anchored in a pelvic bone (14), and a locking portion (7) which corresponds to the transverse passage (4) of the nail (2) in such a way that the locking element (3) can be locked angularly stably with the nail (2), that the nail (2) can be fixed by means of the fixing means at its second nail end in a first pelvic bone (14) and/or the sacrum (15), that the nail is formed in such a way that, after the nail has been fixed at its second end, the pelvic ring can be compressed in the region of the longitudinal extension of the nail by applying a compression pressure to the pelvic ring from outside, that the nail (2) can be fixed by means of the locking element (3) relative to a second pelvic bone in a manner secured against rotation and displacement, and that the nail has a length of 80 mm to 220 mm.

2. Implant (1) according to claim 1, **characterized in that** the nail (2) is designed in such a manner that the nail (2) which can be passed intraosseously through a through-channel pre-drilled through the S1 or S2 corridor (12, 13) of the sacrum (15) has a length of 140 mm to 180 mm and/or that the nail (2) which can be inserted intraosseously into a through-channel pre-drilled in the S1 oder S2 corridor (12, 13) of the sacrum (15) has a length of 80 to 140 mm.

3. Implant (1) according to one of the preceding claims, **characterized in that** the transverse passage (4) has an internal thread which corresponds to a thread provided on the locking portion (7) of the locking element (3) and/or that a bone screw thread is at least provided on the anchoring portion (6, 9) of the locking element (3) and/or that the diameter of the locking element (3) in the locking portion (7) is greater than in the anchoring portion (6).

4. Implant (1) according to one of the preceding claims, **characterized in that** the anchoring portion (6) is arranged on a first end of the locking element (11), the locking portion (7) being arranged behind the anchoring portion (6) as seen from the first end of the locking element (11), a portion of the locking element (3) being arranged behind the locking portion (7) as seen correspondingly, which portion is of pin-like design and whose diameter is at least as large as the outer diameter of the locking element (3) in the locking portion (7).

5. Implant (1) according to one of the preceding claims, **characterized in that** at least one of the locking elements (3) has a second anchoring portion (9), the locking portion (7) being arranged between the first and the second anchoring portions (6, 9), and the second anchoring portion (9) being of a bone-thread type.

6. Implant (1) according to claim 8, **characterized in that** the diameter of the locking element (3) in the second anchoring portion (9) is greater than the diameter of the locking element (3) in the first anchoring portion (6).

7. Implant (1) according to one of the preceding claims, **characterized by** at least two locking elements (3), the nail (2) having at least two transverse passages (4) for receiving a respective one of the locking elements (3), the fixation means for fixing the nail (2) on the second nail end comprising one of the at least two locking elements (3), a distance which at least amounts to 70% of the length of the nail (2) being provided at least between two of the transverse passages (4).

8. Implant (1) according to one of the preceding claims, **characterized in that** at least one or all of the transverse passages (4) has a direction of extension through the nail (2) which forms an angle of greater than 0° to the perpendicular to the axis of extension of the nail (2).

9. Implant (1) according to one of the preceding claims, **characterized in that** the nail (2) includes at least two transverse passages (4), the direction of extension of the first transverse passage (4) forming an angle of greater than 0° with the direction of extension of the second transverse passage (4) in the direction of the axis of extension of the nail (2) and/or in the direction of the perpendicular to the axis of extension of the nail (2).

10. Implant (1) according to claim 9, **characterized in that** the direction of extension of the first transverse passage (2) forms an angle between 5° and 175° with the direction of extension of the second transverse passage (2) in the direction of the axis of extension of the nail (2), the passages (2) on the side of the nail (2) from which the locking elements (3) can be inserted into the passages (2) being less distant from each other than on the opposite side of the nail (2).

11. Locking element (3) for use in an implant (1) according to one of the claims 1 to 10, **characterized by** an anchoring portion (6) and a locking portion (7), the anchoring portion (6) being arranged on a first end of the locking element (11) and the locking element (3) being anchorable with a pelvic bone (14) and/or sacrum (15) by means of the anchoring portion, the locking portion (7) being arranged behind the anchoring portion (6) seen from the first end of the locking element (11), the locking element (7) having an external thread, a portion of pin-like design and having a diameter which is as large as the external diameter of the locking element (3) in the locking portion (7) being arranged behind the locking portion (7), seen from said first end.

12. Locking element (3) according to claim 11, **characterized in that** the diameter of the locking element (3) in the second anchoring portion (9) is greater than the diameter of the locking element (3) in the first anchoring portion (6) and/or in the locking portion (7).

13. Locking element (3) according to one of the claims 11 or 12, **characterized by** a connecting element (16) on the second end thereof for coupling the locking element (3) to a stabilizing assembly (17) of the lower lumbar vertebrae bodies.

14. Locking element (3) according to one of the claims 11 to 13, **characterized in that** the locking element (3) is pierced axially and/or that the locking element (3) has at least one cross bore.

15. Nail (2) for use in an implant (1) for stabilizing pelvic ring fractures in a pelvic ring according to one of the claims 1 to 10, **characterized in that** the nail (2) has a diameter of 7 mm to 10 mm and a length of 80 mm to 220 mm and that the nail (2) has at least one transverse passage (4) at its first nail end for receiving a corresponding locking element, the nail (2) being formed in such a manner that it can be inserted with its second nail end intraosseously into the sacrum (15) via a through-channel pre-drilled in the S1 or S2 corridor (12, 13) of the sacrum (15) and fixed in the sacrum (15) or can be passed through a through-channel pre-drilled through the S1 or S2 corridor (12, 13) of the sacrum (15) and fixed in a first pelvic bone (14) without ensuring fixation of the pelvic ring fracture in the longitudinal direction of the nail, and then a force in the longitudinal direction of the nail is exertable on the pelvic ring from outside under compression of the pelvic ring in the region of the longitudinal direction of the nail for lateral pressurization, the nail (2) being fixable by means of the locking element (3) relative to a second pelvic bone in a manner secured against rotation and displacement while angularly stably maintaining the force on the pelvis ring in the region of the longitudinal extension of the nail.

16. Nail (2) according to claim 15, **characterized in that** the nail (2) has at least two transverse passages (4), a distance which amounts to at least 70% of the length of the nail (2) being provided between the transverse passages (4), and/or that the nail (2) is pierced axially.

17. Nail (2) according to one of the claims 15 or 16, **characterized in that** the nail (2) has a coupling device for coupling a compression ferrule and/or a target device at least to one of its two ends.

## Revendications

1. Implant (1) pour stabiliser les fractures de l'anneau pelvien dans un anneau pelvien, comprenant un clou (2), un moyen de fixation et au moins un élément de verrouillage (3) pour la fixation relative des os pelviens par rapport au clou (2), **caractérisé en ce que** le clou (2) est conçu pour une implantation intra-osseuse et que l'on peut faire traverser par voie intra-osseuse un canal de traversée pré-percé dans le corridor S1 ou S2 (12, 13) du sacrum ou l'on peut introduire par voie intra-osseuse dans un canal de traversée pré-percé dans le corridor S1 ou S2 (12, 13) du sacrum, **en ce que** le clou (2) présente, au moins à sa première extrémité de clou, un passage transversal (4) pour recevoir l'élément de verrouillage (3), **en ce que** l'élément de verrouillage (3) présente une section d'ancrage (6, 9) au moyen de laquelle il peut être fixé dans un os pelvien (14), et une section de verrouillage (7) qui correspond au passage transversal (4) du clou (2) de telle sorte que l'élément de verrouillage (3) peut être verrouillé angulairement stable avec le clou (2), **en ce que** le clou (2) peut être fixé au moyen du moyen de fixation à sa deuxième extrémité de clou dans un premier os pelvien (14) et/ou le sacrum (15), **en ce que** le clou est formé de telle manière que, après la fixation du clou à sa deuxième extrémité, l'anneau pelvien peut être comprimé de l'extérieur dans la région de l'extension longitudinale du clou en appliquant une pression de compression à l'anneau pelvien de l'extérieur, **en ce que** le clou (2) peut être fixé au moyen de l'élément de verrouillage (3) par rapport à un deuxième os pelvien d'une manière fixée contre la rotation et le déplacement, et **en ce que** le clou a une longueur de 80 mm à 220 mm.

2. Implant (1) selon la revendication 1, **caractérisé en ce que** le clou (2) est conçu de telle sorte que le clou (2) qui l'on peut faire traverser par voie intra-osseuse un canal de traversée pré-percé dans le corridor S1 ou S2 (12, 13) du sacrum (15) a une longueur de 140 mm à 180 mm et/ou **en ce que** le clou (2) qui peut être introduit par voie intra-osseuse dans un canal de traversée qui est pré-percé dans le corridor S1 ou S2 (12, 13) du sacrum (15) a une longueur de 80 à 140 mm.

3. Implant (1) selon l'une des revendications précédentes, **caractérisé en ce que** le passage transversal (4) présente un filetage intérieur correspondant à un filetage prévu sur le section de verrouillage (7) de l'élément de verrouillage (3) et/ou **en ce qu'**un filetage de vis à os est prévu au moins sur la section d'ancrage (6, 9) de l'élément de verrouillage (3) et/ou **en ce que** le diamètre de l'élément de verrouillage (3) dans la section de verrouillage (7) est plus grand que dans la section d'ancrage (6).

4. Implant (1) selon l'une des revendications précédentes, **caractérisé en ce que** la section d'ancrage (6) est disposée à une première extrémité de l'élément de verrouillage (11), la section de verrouillage (7) étant disposée derrière la section d'ancrage (6) vue de la première extrémité de l'élément de verrouillage (11), une partie de l'élément de verrouillage (3) étant disposée derrière la section de verrouillage (7), vue de manière correspondante, laquelle partie est en forme de tenon et son diamètre est au moins aussi grand que le diamètre extérieur de l'élément de verrouillage (3) dans la partie de verrouillage (7).

5. Implant (1) selon l'une des revendications précédentes, **caractérisé en ce que** l'un au moins des éléments de verrouillage (3) présente une deuxième section d'ancrage (9), la section de verrouillage (7) étant disposée entre la première et la deuxième section d'ancrage (6, 9) et la deuxième section d'ancrage (9) étant formée à la manière d'un filetage osseux.

6. Implant (1) selon la revendication 8, **caractérisé en ce que** le diamètre de l'élément de verrouillage (3) dans la deuxième section d'ancrage (9) est plus grand que le diamètre de l'élément der verrouillage (3) dans la première section d'ancrage (6).

7. Implant (1) selon l'une des revendications précédentes, **caractérisé par** au moins deux éléments de verrouillage (3), le clou (2) présentant au moins deux passages transversales (4) pour recevoir un élément de verrouillage (3) à la fois, le moyen de fixation comporte l'un des au moins deux éléments de verrouillage (3) pour la fixation du clou (2) à sa deuxième extrémité, une distance d'au moins 70 % de la longueur du clou (2) étant prévue entre au moins deux passages transversaux (4).

8. Implant (1) selon l'une des revendications précédentes, **caractérisé en ce qu'**au moins un ou tous les passages transversaux (4) présentent une direction d'extension à travers le clou (2) formant un angle supérieur à 0° par rapport à la perpendiculaire à la direction d'extension du clou (2).

9. Implant (1) selon l'une des revendications précédentes, caractérisé en ce le clou (2) présente au moins deux passages transversaux (4), la direction d'extension du premier passage transversal (4) formant un angle supérieur à 0° avec la direction d'extension du deuxième passage transversal (4) dans la direction de l'axe d'extension du clou (2) et/ou dans la direction perpendiculaire à l'axe d'extension du clou (2).

10. Implant (1) selon la revendication 9, **caractérisé en ce que** la direction d'extension du premier passage transversal (2) forme un angle entre 5° et 175° avec la direction d'extension du deuxième passage transversal (2) dans la direction de l'axe d'extension du clou (2), lesdits passages (2) sur le côté du clou (2) à partir duquel les éléments de verrouillage (3) peuvent être insérés dans les bagues (2) étant moins éloignés les uns des autres que sur le côté opposé du clou (2).

11. Élément de verrouillage (3) pour l'utilisation dans un implant (1) selon l'une quelconque des revendications 1 à 10, **caractérisé par** une section d'ancrage (6) et une section de verrouillage (7), la section d'ancrage (6) étant disposée à une première extrémité de l'élément de verrouillage (11) et l'élément der verrouillage (3) pouvant être ancré avec un os pelvien (14) et/ou sacrum (15) au moyen de la section d'ancrage (6), la section de verrouillage (7) étant disposée derrière la section d'ancrage (6), vue de la première extrémité de l'élément de verrouillage (11), la section de verrouillage (7) présentant un filetage extérieur, une partie en forme de tenon et dont le diamètre est au moins égal au diamètre extérieur de l'élément de verrouillage (3) dans la section de verrouillage (7) étant disposée derrière la section de verrouillage (7), vue de la première extrémité.

12. Élément de verrouillage (3) selon la revendication 11, **caractérisé en ce que** le diamètre de l'élément de verrouillage (3) dans la deuxième section d'ancrage (9) est plus grand que le diamètre de l'élément de verrouillage (3) dans la première section d'ancrage (6) et/ou dans la section de verrouillage (7).

13. Élément de verrouillage (3) selon l'une des revendications 11 ou 12, **caractérisé par** un élément de liaison à sa deuxième extrémité pour l'accouplement de l'élément de verrouillage (3) à un montage de stabilisation (17) des vertèbres lombaires inférieures.

14. Élément der verrouillage (3) selon l'une des revendications 11 à 13, **caractérisé en ce que** l'élément de verrouillage (3) est percé axialement et/ou l'élément de verrouillage (3) présente au moins un alésage transversal.

15. Clou (2) pour l'utilisation dans un implant (1) pour stabiliser les fractures de l'anneau pelvien dans un anneau pelvien selon l'une des revendications 1 à 10, **caractérisé en ce que** le clou (2) a un diamètre de 7 mm à 10 mm et une longueur de 80 mm à 220 mm et **en ce que** le clou (2) présente au moins un passage transversal (4) à sa première extrémité de clou pour recevoir un élément de verrouillage correspondant, le clou (2) étant formé de telle manière qu'il peut être introduit avec sa deuxième extrémité de clou par voie intra-osseuse dans le sacrum (15) à travers un canal de traversée pré-percé dans un corridor S1 ou S2 (12, 13) du sacrum (15) et peut être fixé dans le sacrum (15) ou peut passer par un canal de traversée pré-percé dans le corridor S1 ou S2 (12, 13) du sacrum (15) et peut être fixé dans un premier os pelvien (14), sans assurer une fixation de la fracture de l'anneau pelvien dans la direction longitudinale du clou, puis une force dans la direction longitudinale du clou peut être exercée sur l'anneau pelvien de l'extérieur, en compression latérale dans la zone de l'extension longitudinale du clou, le clou (2) pouvant être fixé au moyen de l'élément de verrouillage (3) par rapport à un deuxième os pelvien d'une manière fixée contre la rotation et le déplacement, tout en maintenant la stabilité angulaire de la force sur l'anneau pelvien dans la zone de l'extension longitudinale du clou.

16. Clou (2) selon la revendication 15, **caractérisé en ce que** le clou (2) présente au moins deux passages transversaux (4), une distance d'au moins 70 % de la longueur du clou (2) étant prévue entre au moins deux passages transversaux (4), et/ou **en ce que** le clou (2) est percé axialement.

17. Clou (2) selon l'une des revendications 15 ou 16, **caractérisé en ce que** le clou (2) présente, au moins à l'une de ses deux extrémités, un dispositif d'accouplement pour accoupler une pince de compression et/ou un dispositif cible.
